# EUROPEAN PATENT APPLICATION

(11) **EP 1 477 910 A1**
(43) Date of publication of application: **17.11.2004**
(21) Application number: 03705276.8
(22) Date of filing: 18.02.2003
(51) Int. Cl.: G06F 17/30, C12N 1/00, C12N 15/09

(54) **APPARATUS FOR MANAGING GENE EXPRESSION DATA**

(30) Priority: 18.02.2002 JP 2002040746; 06.09.2002 JP 2002262012
(71) Applicant: Celestar Lexico-Sciences, Inc., Chiba-shi, Chiba 261-8501 (JP)
(72) Inventor: UEMURA, Yasuo, c/o Celestar Lexico-Sciences, Inc., Chiba-shi, Chiba 261-8501 (JP); DOI, Hirofumi, c/o Celestar Lexico-Sciences, Inc., Chiba-shi, Chiba 261-8501 (JP); KAWAMURA, Akira, Celestar Lexico-Sciences, Inc., Chiba-shi, Chiba 261-8501 (JP)
(74) Representative: HOFFMANN - EITLE
(86) International application number: PCT/JP2003/001708
(87) International publication number: WO 2003/081471

(57) **Abstract**

Genetic clones aligned on a multiwell plate are produced and a collection of genes expressed in a specific tissue are produced. Nucleotide sequences of genetic clones are determined. An analysis is performed based on the nucleotide sequences. An in situ hybridization experiment is conducted, and expression statuses of genes that correspond to the genetic clones in a specific cell, tissue or organ are examined.

## Description

### TECHNICAL FIELD

The present invention relates to a gene expression information management system, a gene expression information management method, and a gene expression information management program. More specifically, the present invention relates to a gene expression information management system, a gene expression information management method, and a computer program for managing photomicroscopic images of gene expression analysis.

The present invention also relates to an in situ hybridization analysis management method and an in situ hybridization analysis management system which can generally manage image information and gene-related information acquired by various gene expression analysis and which can extract knowledges in full.

### BACKGROUND ART

For decades, in situ hybridization analysis has been widely carried out to identify positional distribution or localization of expressed genes or proteins within cells or tissues of interest by directly hybridizing the probe of a specific gene to the histologically intact cell or tissue and then making observations with optical microscope or electron microscope.

In addition, a large-scale in situ hybridization method for tissue section is developed by KOMIYA, Toru from Japan Science and Technology Corporation "Doi Bio-Asymmetry Project" (see KOMIYA, Toru, "96 wells de okonau seppen no in situ hybridization (In situ Hybridization to Tissue Section on 96-well plate)", Saibo Kogaku 18,405, 1999; and KOMIYA, Toru, "In situ hybridization ni yoru hatsugen chizu (Expression Map by in situ Hybridization)", Genome Kino Hatsugen Profile to Transcriptome (Functional Genomics and Transcriptome), Nakayama Shoten, Co., Ltd., pp. 102-115, 2000, and the like.)

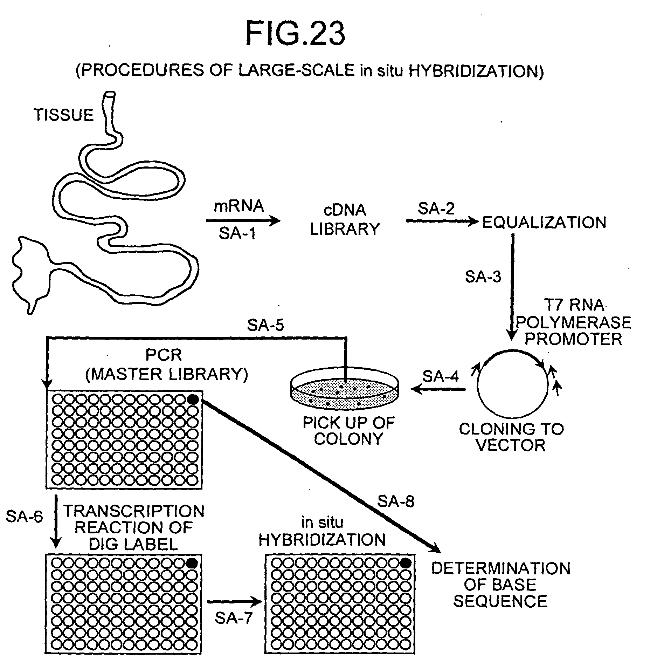

The outline of this large-scale in situ hybridization method will be explained with reference to Fig. 23. As shown in Fig. 23, mRNA is extracted from an organic tissue or the like, cDNA is synthesized from mRNA using Notl oligo (dT) primer, and cDNA library is constructed (at a step SA-1). The cDNA library is equalized to thereby provide an equalized cDNA library (at a step SA -2). According to this method, even the mRNA of extremely small copy numbers can be identified using the equalized cDNA library.

The ligation reaction of cDNA with a vector is carried out to transform cDNA into Escherichia coli (at a step SA -3). A colony of transformed E. coli is created (at a step SA -4). The colonies are picked up at random, and insert cDNA is linearized and amplified by PCR using vector sequence primers. At that time, the promoter sequence of RNA polymerase derived from the vector is added to the cDNA fragments. The amplified cDNA fragments are purified on a 96-well plate using a glass powder method, and stored as a master library (at a step SA -5).

Using purified fragments, a DIG (digoxigenin) label serving as a hapten, is subjected to a transcription reaction (at a step SA -6). A probe obtained is purified by ethanol precipitation, dissolved, and hybridized in situ with a tissue section fixed at the bottom of the 96-well plate (at a step SA -7). Washing after hybridization, enzyme coupled anti-DIG antibody reaction is systematically controlled using an ELISA plate washer.

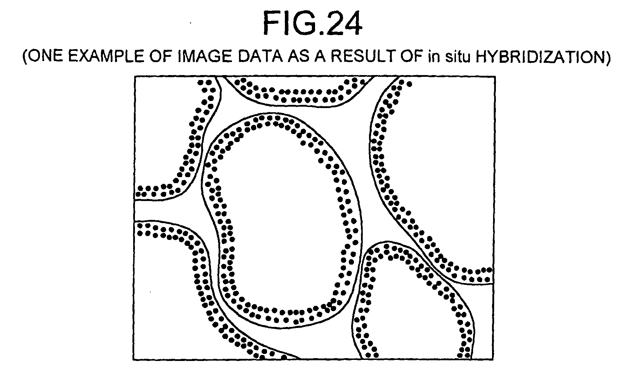

After color development by an enzyme labeled antibody.method, the section is observed with an inverted microscope, and photographed by a CCD camera. Fig. 24 illustrates an example of the photographed image. If an image shows an intriguing signal of expression (indicated by black in Fig. 24), the nucleotide sequences of the corresponding clone in the master library are determined. Thus, the sequence-related information can be combined with the gene expression image (at a step SA-8).

According to the conventional method, however, the acquisition of the expression images by the in situ hybridization, matching the images with the base nucleotide sequences of the cDNA clones used as probes, and discovery of biological knowledges based on those information has been manually labored task. Therefore, the conventional method is disadvantageous in that it is difficult to generally manage the obtained information and extract knowledges in full.

These disadvantages will be explained in detail below.

In the large-scale in situ hybridization, large quantities of expression images thus obtained, a homology search for genetic sequences thereof, and the like are analyzed manually. Namely, an experimenter needs to search an existing cDNA DB (eg., an expressed sequence tag (hereinafter, "EST") DB or a full-length cDNA DB) for similar sequences using a homology search program such as BLAST based on the base sequences of the expressed cDNA clones, and identify an expressed gene (mRNA). In addition, the experimenter needs to collect and do clustering cDNA sequences so that clones representing the identical mRNA should be grouped into the single cluster, group expression images for the clones in the same cluster, and extract knowledges read from the images.

Furthermore, if such a large-scale in situ hybridization experiment is conducted for one tissue, at least several hundreds to several thousands of images are acquired. Besides, it is expected that experimental data for the expression analysis by means of the large-scale in situ hybridization or the like increases enormously following the recent development of transcriptome analysis and proteome analysis. Naturally, therefore, there is a limit to manual operations, and the development of a database system that can generally manage the operations is desired, accordingly.

Moreover, if the database system which can generally manage the experimental data on the in situ hybridization is introduced and a data mining method is utilized, it is expected to be able to discover knowledges, which have not been discovered by the conventional manual analysis, on a computer system.

In addition, if an experiment is conducted using many plates, progress management per plate as to what processing is performed for which plate, a processing is to be stopped for which plate, and the like is complicated, whereby unnatural errors disadvantageously occur.

The conventional techniques explained above and objects that the present invention is to attain are not limited to an instance of the collection of experimental data by means of the large-scale in situ hybridization but similarly apply to all other experimental methods for analyzing gene expression information.

It is, therefore, an object of the present invention to provide an in situ hybridization analysis management method and an in situ hybridization analysis management system which can generally manage image information and gene-related information acquired by various gene expression analysis and which can extract knowledges in full.

### DISCLOSURE OF THE INVENTION

It is an object of the present invention to solve at least the problems in the conventional technology. A gene expression information management apparatus according to one aspect of the present invention comprises an image data input unit which inputs pieces of image data on expression of genes; a base sequence input unit which inputs base sequences of the expressed genes; a homology search unit which conducts a homology search of the base sequences input by the base sequence input unit, and which extracts homologous sequences; and a display unit which displays the image data, the base sequences corresponding to the image data, and the homologous sequences.

This apparatus inputs image data on the expression of genes, inputs base sequences of the expressed genes (e.g., base sequences of cDNA clones), conducts a homology search of the input sequences to extract homologous sequences; and displays the image data, the corresponding sequences and the homologous sequences. Therefore, the genes expressed in the image data can be easily specified.

That is, a homology search (e.g., FastA or Blast) is conducted to known sequences stored in a sequence database (e.g., an EST database or a full-length cDNA database) for the base sequences of cDNA clones used as probes and corresponding images picked up at a gene expression analysis such as an in situ hybridization experiment, and base sequences having high similarities are displayed. Thus, the cDNA can be specified, and both the cDNA and the image data can be displayed comprehensively.

Further, a similarity (e.g., the score of the homology search) between each base sequence and each of its homologous sequences may be displayed together with the homologous sequence. Thus, the most similar sequence can be displayed. In addition, the homologous sequences can be displayed while being sorted by homology score.

Furthermore, at least one of information attached to each homologous sequence, i.e., the name of a gene, the name of the protein product of the gene, an organism from which the gene is acquired, the name of an organ or a tissue from which the gene is acquired, the ID of the gene in the GenBank, the ID of the protein product of the gene in the GenBank, the length and the similarity by which the sequence of the cDNA is matched with the sequence of the gene, and information on the evidence of the presence of the gene may be displayed together with the homologous sequence.

In the gene expression information management apparatus, the homology search unit conducts the homology search for a base sequence of at least one of:
(1) a gene which is known in the same or an other organism;
(2) a gene which is unknown but a cDNA of which is already acquired;
(3) a gene which is unknown but a corresponding genome DNA of which is already acquired;
(4) a gene whose location on a chromosome is known; and
(5) a gene which is already patented.

This illustrates one example of the homology search more concretely. This apparatus conducts the homology search for a base sequence of at least one of: (1) a gene which is known to the same or an other organism; (2) a gene which is unknown but a cDNA of which is already acquired; (3) a gene which is unknown but a corresponding genome DNA of which is already acquired; (4) a gene whose location on a chromosome is known; and (5) a gene which is already patented. Therefore, the biological significance of the image data or the like can be easily specified.

That is, since the apparatus can conduct a homology search to the respective sequence databases which store base sequences in the categories (1) to (5), the most homologous sequence in each category can be individually specified.

A gene expression information management apparatus according to another aspect of the present invention comprises an image data input unit which inputs pieces of image data on expression of genes; a base sequence input unit which inputs base sequences of the expressed genes; a sequence clustering unit which clusters the base sequences input by the base sequence input unit, and which classifies the base sequences into specific clusters; and a display unit which displays the image data and the base sequences corresponding to the image data for each of the clusters.

This apparatus can input image data on the expression of genes, input base sequences of the expressed genes (e.g., base sequences of cDNA clones), cluster the input base sequences to classify the base sequences into specific clusters, and displays the image data, the corresponding sequences, and the homologous sequences to the corresponding sequences for each cluster. Therefore, by classifying, for example, cDNA (EST sequences) derived from the same mRNA into the same cluster, the base sequences having the same property can be collected and classified into the specific cluster.

This makes the elimination or the like of the duplication of the cDNA clones extracted at random from the cDNA library possible, thereby further improving efficiency for an operation for generalizing experimental results.

The gene expression information management apparatus further comprises a cluster sequencing unit which determines a cluster sequence from the base sequences classified into the same cluster by the sequence clustering unit, wherein the display unit displays the cluster sequence, the image data, and the base sequences corresponding to the image data for each of the clusters.

This apparatus determines a cluster sequence from the base sequences classified into the same cluster, and displays the cluster sequence, the image data, and the corresponding base sequences for each cluster. Therefore, a base sequence (e.g., a full-length cDNA) created by combining the base sequences belonging to the same cluster can be determined as the cluster sequence and displayed.

This can facilitate predicting a genetic sequence such as a full-length DNA (mRNA sequence) from an experiment using partial cDNA sequences (EST sequences).

In the gene expression information management apparatus, the sequence clustering unit assembles the base sequences into a consensus sequence, and classifies the base sequences constituting the same consensus sequence into the same cluster, and the sequence clustering unit determines the consensus sequence of the cluster as the cluster sequence.

This apparatus assembles the base sequences into a consensus sequence, classifies the base sequences constituting the same consensus sequence into the same cluster, and determines the consensus sequence of the cluster as the cluster sequence. Therefore, a cDNA sequence close to a full-length cDNA sequence can be created from partial cDNA sequences using a sequence assembly technique (for creating a long sequence from short sequence fragments. For example, an overlap between sequence fragments is searched by a multiple sequence alignment method or the like, and the sequence fragments having the overlap are synthesized, whereby a longer sequence is created.)

The gene expression information management apparatus further comprises a cluster sequence homology search unit which conducts a homology search of the cluster sequence determined by the cluster sequencing unit, and which extracts homologous sequences. The display unit displays the cluster sequence, the homologous sequence to the cluster sequence, the image data, and the corresponding sequences for each of the cluster.

This apparatus conducts a homology search of the determined cluster sequence to extract homologous sequences, and displays the cluster sequence, the homologous sequence to the cluster sequence, the image data, and the corresponding sequences for each of the cluster. This can facilitate specifying the expressed genes in the image data.

That is, a homology search (e.g., FastA or Blast) is conducted to known sequences stored in the sequence database (e.g., the EST database or the full-length cDNA database) for the cluster sequence synthesized from the base sequences of the cDNA clones used as probes by executing a sequence assembly processing or the like, and base sequences having high similarities are displayed. Thus, the expressed genes can be easily specified.

Further, a similarity (e.g., the score of the homology search) between each base sequence and each of its homologous sequences may be displayed together with the homologous sequence. Thus, the most similar sequence to this cluster sequence can be displayed. In addition, the homologous sequences can be displayed while being sorted by homology score.

Furthermore, at least one of information attached to each homologous sequence, i.e., the name of a gene, the name of the protein product of the gene, an organism from which the gene is acquired, the name of an organ or a tissue from which the gene is acquired, the ID of the gene in the GenBank, the ID of the protein product of the gene in the GenBank, the length and the similarity by which the cluster sequence is matched with the sequence of the gene, and information on the evidence of the presence of the gene may be displayed together with the homologous sequence.

Furthermore, if arbitrary genetic sequences are input and clustered with registered cDNA sequences, information (e.g., image data) on the cluster sequences belonging to the same cluster can be displayed.

In the gene expression information management apparatus, the cluster sequence homology search unit conducts the homology search for a base sequence of at least one of:
(1) a gene which is known in the same or an other organism;
(2) a gene which is unknown but a cDNA of which is already acquired;
(3) a gene which is unknown but a corresponding genome DNA of which is already acquired;
(4) a gene whose location on a chromosome is known; and
(5) a gene which is already patented.

This illustrates one example of the homology search more concretely. This apparatus conducts the homology search for a base sequence of at least one of: (1) a gene which is known to the same or an other organism; (2) a gene which is unknown but a cDNA of which is already acquired; (3) a gene which is unknown but a corresponding genome DNA of which is already acquired; (4) a gene whose location on a chromosome is known; and (5) a gene which is already patented. Therefore, the biological significance of the image data or the like can be easily specified.

That is, since the apparatus can conduct a homology search to the respective sequence databases which store base sequences in the categories (1) to (5), the most homologous sequence in each category can be individually specified.

The gene expression information management apparatus, further comprises an annotation information storage unit which stores at least one of information on an extracted tissue, information on a growth stage or an ageing stage of the extracted tissue, information as to whether gene expression is observed, and information on a region in which the gene expression is observed while making the at least one information correspond to the image data, wherein the display unit displays at least one of the information on the extracted tissue, the information on the growth stage or the ageing stage of the extracted tissue, the information as to whether the gene expression is observed, and the information on the region in which the gene expression is observed while making the at least one information correspond to the image data.

This illustrates one example of the annotation information on the image data more concretely. This apparatus can store at least one of information on an extracted tissue, information on a growth stage or an ageing stage of the extracted tissue, information as to whether gene expression is observed, and information on a region in which the gene expression is observed while making the at least one information correspond to the image data, and display at least one of the information on the extracted tissue, the information on the growth stage or the ageing stage of the extracted tissue, the information as to whether the gene expression is observed, and the information on the region in which the gene expression is observed while making the at least one information correspond to the image data.

The gene expression information management apparatus further comprises an expression level estimation unit which estimates expression levels of the genes in the image data based on one of or both of the image data and the base sequences.

This apparatus estimates expression levels of the genes in the image data based on one of or both of the image data and the base sequences. This can facilitate specifying an expression pattern (a pattern of uniform expression, non-uniform expression or the like).

The expression levels may be estimated by obtaining the signal intensity and the area of signal region of a fluorescent dye or the like in the image data by means of a known image analysis method or the like. Further, by using information on not only the image data but also the base sequences, an automatic estimation can be made as follows. If a genomic repeat sequence, for example, is included in the base sequences, the probability of cross-hybridization (occurrence of a hybridization reaction to other mRNA having the same genomic repeat sequence) is high. Therefore, the reliability of the estimated expression level is low.

Moreover, if images of the same tissue section in which the probes are made illuminant and in which the probes are not made illuminant are picked up or images of the same tissue section which are colored with different fluorescent dyes are picked up, the image analysis processing can be easily executed (by, for example, estimating an expression level by obtaining the difference between the two images).

The gene expression information management apparatus further comprises an expression level order sorting unit which sorts display orders of the image data according to the expression levels estimated by the expression level estimation unit.

Since this apparatus sorts display orders of the image data according to the estimated expression levels, the user can efficiently check the experimental result.

The gene expression information management apparatus further comprises an image comparison unit which compares two or more pieces of the image data based on at least one of the image data, the base sequences, the expression levels, the information on the extracted tissue, the information on the growth stage or the ageing stage of the extracted tissue, the information as to whether the gene expression is observed, and the information on the region in which the gene expression is observed; and a difference extraction unit which extracts a difference among the two or more pieces of the image data based on a comparison result of the image comparison unit.

This apparatus compares two or more pieces of the image data based on at least one of the image data, the base sequences, the expression levels, the information on the extracted tissue, the information on the growth stage or the ageing stage of the extracted tissue, the information as to whether the gene expression is observed, and the information on the region in which the gene expression is observed, and extracts a difference among the two or more pieces of the image data based on a comparison result. Therefore, the apparatus can efficiently extract the difference among the images.

Thus, if annotation processings are carried out for the expression patterns in the respective tissues by image recognition or manual operation, and annotation results are automatically compared, the images of the tissues having a difference can be extracted and displayed.

Further, the comparison of, for example, a normal cell with a disease cell, that of the growth stage or ageing stages of the cells at time series, and that of before medication with after medication or the like can be efficiently executed.

The gene expression information management apparatus further comprises a three-dimensional image creation unit which creates a three-dimensional image from two or more pieces of the image data; and an expression level simulation unit which simulates expression levels in the three-dimensional image from the expression levels in the image data.

This apparatus creates a three-dimensional image from two or more pieces of the image data, and simulates expression levels in the three-dimensional image from the expression levels in the image data. Therefore, if slices of an organ are all tested based on one sequence, the three-dimensional image of the organ can be simulated by combining the slice images and the expression level of an mRNA obtained by analyzing each image can be corrected three-dimensionally and displayed.

The gene expression information management apparatus further comprises a typical clone determination unit which determines a typical clone from the base sequences belonging to the same cluster, based on at least one of the image data, the base sequences, the expression levels, the information on the extracted tissue, the information on the growth stage or the ageing stage of the extracted tissue, the information as to whether the gene expression is observed, and the information on the region in which the gene expression is observed.

This apparatus determines a typical clone from the base sequences belonging to the same cluster, based on at least one of the image data, the base sequences, the expression levels, the information on the extracted tissue, the information on the growth stage or the ageing stage of the extracted tissue, the information as to whether the gene expression is observed, and the information on the region in which the gene expression is observed. Therefore, a clone which can be expected to provide the best experimental data can be selected from among the clones derived from the same mRNA and extracted as a typical clone. In addition, EST clones considered to be derived from the same mRNA are classified into the same cluster and an experiment is conducted only to a typical clone in the cluster, whereby the number of experiments can be decreased. In other words, while as many experiments as EST clones have been conventionally required, the sequence clustering enables only the typical clones (as many as the number of clusters) to be experimented.

However, even if the cDNA clones are derived from the same mRNA, sequences thereof slightly differ depending on cut portions. If the in situ hybridization is carried out, some cDNA clones emit fine expression signals and others do not. Due to this, the typical clone may be determined by observing image data and selecting the clone which emits a good expression signal.

Alternatively, the cDNA clone having a base sequence which does not include a genomic repeat sequence or having a sequence length suitable for the experiments may be selected as a typical clone.

The gene expression information management apparatus further comprises a cluster significance determination unit which determines a significance of each of the clusters based on at least one of a cluster sequence homology search result, the image data, the base sequences, the expression levels, the information on the extracted tissue, the information on the growth stage or the ageing stage of the extracted tissue, the information as to whether the gene expression is observed, and the information on the region in which the gene expression is observed.

This apparatus determines the significance of each of the clusters based on at least one of a cluster sequence homology search result, the image data, the base sequences, the expression levels, the information on the extracted tissue, the information on the growth stage or the ageing stage of the extracted tissue, the information as to whether the gene expression is observed, and the information on the region in which the gene expression is observed. Therefore, based on the information, the significance of each cluster can be arbitrarily determined and the cluster which interests the user can be easily discovered.

For example, the significance of a clone which shows a high expression level in a tissue in a specific growth stage or ageing stage can be set high based on information on expression levels and the tissue. Further, If the result of a homology search to the base sequence indicates that there is no hit clone in the existing genetic sequence DB (i.e., a known homologous sequence is not present in the DB), the significance can be set higher.

The gene expression information management apparatus further comprises a genetic locus specification unit which specifies a genetic locus on a chromosome in which the base sequences are present; a chromosome map creation unit which creates a chromosome map by mapping information on the base sequences on the genetic locus of the chromosome; and a chromosome map display unit which displays the chromosome map created by the chromosome map creation unit.

This apparatus can specify a genetic locus on a chromosome in which the base sequences are present, and create a chromosome map by mapping information (e.g., image data, base sequences, expression levels, information on an extracted tissue, information on the growth stage or ageing stage of the extracted tissue, information as to whether gene expression is observed, and information on a region in which the gene expression is observed) on the base sequences on the genetic locus of the chromosome.

In addition, the apparatus may be made to display detailed information on the base sequence by selecting a portion of the chromosome map corresponding to the genetic locus (which portion may be indicated by a specific mark).

A gene expression information management method according to another aspect of the present invention comprises an image data input step of inputting pieces of image data on expression of genes; a base sequence input step of inputting base sequences of the expressed genes; a homology search step of conducting a homology search of the base sequences input at the base sequence input step, and extracting homologous sequences; and a display step of displaying the image data, the base sequences corresponding to the image data, and the homologous sequences.

According to this method, image data on the expression of genes is input, base sequences of the expressed genes (e.g., base sequences of cDNA clones) is input, a homology search is conducted to the input sequences to extract homologous sequences, and the image data, the corresponding sequences and the homologous sequences are displayed. Therefore, the genes expressed in the image data can be easily specified.

That is, a homology search (e.g., FastA or Blast) is conducted to known sequences stored in a sequence database (e.g., an EST database or a full-length cDNA database) for the base sequences of cDNA clones used as probes and corresponding images picked up at a gene expression anylysis such as an in situ hybridization experiment, and base sequences having high similarities are displayed. Thus, the cDNA can be specified, and both the cDNA and the image data can be displayed comprehensively.

Further, a similarity (e.g., the score of the homology search) between each base sequence and each of its homologous sequences may be displayed together with the homologous. Thus, the most similar sequence can be displayed. In addition, the homologous sequences can be displayed while being sorted by homology score.

Furthermore, at least one of information attached to each homologous sequence, i.e., the name of a gene, the name of the protein product of the gene, an organism from which the gene is acquired, the name of an organ or a tissue from which the gene is acquired, the ID of the gene in the GenBank, the ID of the protein product of the gene in the GenBank, the length and the similarity by which the sequence of the cDNA is matched with the sequence of the gene, and information on the evidence of the presence of the gene may be displayed together with the homologous sequence.

In the gene expression information management method, at the homology search step, the homology search is conducted for a base sequence of at least one of:
(1) a gene which is known in the same or an other organism;
(2) a gene which is unknown but a cDNA of which is already acquired;
(3) a gene which is unknown but a corresponding genome DNA of which is already acquired;
(4) a gene whose location on a chromosome is known; and
(5) a gene which is already patented.

This illustrates one example of the homology search more concretely. According to this method, the homology search is conducted for a base sequence of at least one of: (1) a gene which is known to the same or an other organism; (2) a gene which is unknown but a cDNA of which is already acquired; (3) a gene which is unknown but a corresponding genome DNA of which is already acquired; (4) a gene whose location on a chromosome is known; and (5) a gene which is already patented. Therefore, the biological significance of the image data or the like can be easily specified.

That is, since a homology search can be conducted to the respective sequence databases which store base sequences in the categories (1) to (5), the most homologous sequence in each category can be individually specified.

A gene expression information management method according to still another aspect of the present invention comprises an image data input step of inputting pieces of image data on expression of genes; a base sequence input step of inputting base sequences of the expressed genes; a sequence clustering step of sequence clustering the base sequences input at the base sequence input step, and classifying the base sequences into specific clusters; and a display step of displaying the image data and the base sequences corresponding to the image data for each of the clusters.

According to this method, image data on the expression of genes can be input, base sequences of the expressed genes (e.g., base sequences of cDNA clones) can be input, the input base sequences can be clustered to classify the base sequences into specific clusters, and the image data, the corresponding base sequences, and the homologous sequences to the corresponding base sequences can be displayed for each cluster. Therefore, by classifying, for example, cDNA (EST sequences) derived from the same mRNA into the same cluster, the base sequences having the same property can be collected and classified into the specific cluster.

This makes the elimination or the like of the duplication of the cDNA clones extracted at random from the cDNA library possible, thereby further improving efficiency for an operation for generalizing experimental results.

The gene expression information management method further comprises a cluster sequencing step of determining a cluster sequence from the base sequences classified into the same cluster at the sequence clustering step, wherein at the display step, the cluster sequence and the base sequences corresponding to the image data are displayed for each of the clusters.

According to this method, a cluster sequence is determined from the base sequences classified into the same cluster, and the cluster sequence, the image data, and the corresponding base sequences are displayed for each cluster. Therefore, a base sequence (e.g., a full-length cDNA) created by combining the base sequences belonging to the same cluster can be determined as the cluster sequence and displayed.

This can facilitate predicting a genetic sequence such as a full-length DNA (mRNA sequence) from an experiment using partial cDNA sequences (EST sequences).

In the gene expression information management method, at the sequence clustering step, the base sequences are assembled into a consensus sequence, the base sequences constituting the same consensus sequence are classified into the same cluster, and at the sequence clustering step, the consensus sequence of the cluster is determined as the cluster sequence.

According to this method, the base sequences are assembled into a consensus sequence, the base sequences constituting the same consensus sequence are classified into the same cluster, and the consensus sequence of the cluster is determined as the cluster sequence. Therefore, a cDNA sequence close to a full-length cDNA sequence can be created from partial cDNA sequences using a sequence assembly technique (for creating a long sequence from short sequence fragments. For example, an overlap between sequence fragments is searched by a multiple sequence alignment method or the like, and the sequence fragments having the overlap are synthesized, whereby a longer sequence is created.)

The gene expression information management method further comprises a cluster sequence homology search step of conducting a homology search of the cluster sequence determined at the cluster sequencing step, and extracting homologous sequences. At the display step, the cluster sequence, the homologous sequence to the cluster sequence, and the corresponding sequences are displayed for each of the cluster.

According to this method, a homology search is conducted of the determined cluster sequence to extract homologous sequences, and the cluster sequence, the homologous sequence to the cluster sequence, the image data, and the corresponding base sequences for each of the cluster. This can facilitate specifying the expressed genes in the image data.

That is, a homology search (e.g., FastA or Blast) is conducted to known sequences stored in the sequence database (e:g., the EST database or the full-length cDNA database) for the cluster sequence synthesized from the base sequences of the cDNA clones used as probes by executing a sequence assembly processing or the like, and base sequences having high similarities are displayed. Thus, the expressed genes can be easily specified.

Further, a similarity (e.g., the score of the homology search) between each base sequence and each of its homologous sequences may be displayed together with the homologous sequence. Thus, the most similar sequence to this cluster sequence can be displayed. In addition, the homologous sequences can be displayed while being sorted by homology score.

Furthermore, at least one of information attached to each homologous sequence, i.e., the name of a gene, the name of the protein product of the gene, an organism from which the gene is acquired, the name of an organ or a tissue from which the gene is acquired, the ID of the gene in the GenBank, the ID of the protein product of the gene in the GenBank, the length and the similarity by which the cluster sequence is matched with the sequence of the gene, and information on the evidence of the presence of the gene may be displayed together with the homologous sequence.

Furthermore, if arbitrary genetic sequences are input and clustered with registered cDNA clone sequences, information (e.g., image data) on the cluster sequences belonging to the same cluster can be displayed.

In the gene expression information management method, at the cluster sequence homology search step, the homology search is conducts for a base sequence of at least one of:
(1) a gene which is known in the same or an other organism;
(2) a gene which is unknown but a cDNA of which is already acquired;
(3) a gene which is unknown but a corresponding genome DNA of which is already acquired;
(4) a gene whose location on a chromosome is known; and
(5) a gene which is already patented.

This illustrates one example of the homology search more concretely. According to this method, the homology search is conducted for a base sequence of at least one of: (1) a gene which is known to the same or an other organism; (2) a gene which is unknown but a cDNA of which is already acquired; (3) a gene which is unknown but a corresponding genome DNA of which is already acquired; (4) a gene whose location on a chromosome is known; and (5) a gene which is already patented. Therefore, the biological significance of the image data or the like can be easily specified.

That is, since a homology search can be conducted to the respective sequence databases which store base sequences in the categories (1) to (5), the most homologous sequence in each category can be individually specified.

The gene expression information management method, further comprises an annotation information storage step of storing at least one of information on an extracted tissue, information on a'growth stage or an ageing stage of the extracted tissue, information as to whether gene expression is observed, and information on a region in which the gene expression is observed while making the at least one information correspond to the image data, wherein at the display step, at least one of the information on the extracted tissue, the information on the growth stage or the ageing stage of the extracted tissue, the information as to whether the gene expression is observed, and the information on the region in which the gene expression is observed is displayed while making the at least one information correspond to the image data.

This illustrates one example of the annotation information on the image data more concretely. According to this method, at least one of information on an extracted tissue, information on a growth stage or an ageing stage of the extracted tissue, information as to whether gene expression is observed, and information on a region in which the gene expression is observed can be stored while making the at least one information correspond to the image data, and at least one of the information on the extracted tissue, the information on the growth stage or the ageing stage of the extracted tissue, the information as to whether the gene expression is observed, and the information on the region in which the gene expression is observed can be displayed while making the at least one information correspond to the image data.

The gene expression information management method further comprises an expression level estimation step of estimating expression levels of the genes in the image data based on one of or both of the image data and the base sequences.

According to this method, expression levels of the genes in the image data are estimated based on one of or both of the image data and the base sequences. This can facilitate specifying an expression pattern (a pattern of uniform expression, non-uniform expression or the like).

The expression levels may be estimated by obtaining the signal intensity and the area of signal region of a fluorescent dye or the like in the image data by means of a known image analysis method or the like. Further, by using information on not only the image data but also the base sequences, an automatic estimation can be made as follows. If a genomic repeat sequence, for example, is included in the base sequences, the probability of cross-hybridization (occurrence of a hybridization reaction to other mRNA having the same genomic repeat sequence) is high. Therefore, the reliability of the estimated expression level is low.

Moreover, if images of the same tissue section in which the probes are made illuminant and in which the probes are not made illuminant are picked up or images of the same tissue section which are colored with different fluorescent dyes are picked up, the image analysis processing can be easily executed (by, for example, estimating an expression level by obtaining the difference between the two images).

The gene expression information management method further comprises an expression level order sorting step of sorting display orders of the image data according to the expression levels estimated at the expression level estimation step.

Since it is possible to sort display orders of the image data according to the estimated expression levels according to this method, the user can efficiently check the experimental result.

The gene expression information management method further comprises an image comparison step of comparing two or more pieces of the image data based on at least one of the image data, the base sequences, the expression levels, the information on the extracted tissue, the information on the growth stage or the ageing stage of the extracted tissue, the information as to whether the gene expression is observed, and the information on the region in which the gene expression is observed; and a difference extraction step of extracting a difference among the two or more pieces of the image data based on a comparison result of the image comparison step.

According to this method, two or more pieces of the image data are compared based on at least one of the image data, the base sequences, the expression levels, the information on the extracted tissue, the information on the growth stage or the ageing stage of the extracted tissue, the information as to whether the gene expression is observed, and the information on the region in which the gene expression is observed, and a difference among the two or more pieces of the image data is extracted based on a comparison result. Therefore, it is possible to efficiently extract the difference among the images.

Thus, if annotation processings are carried out'for the expression patterns in the respective tissues by image recognition or manual operation, and annotation results are automatically compared, the images of the tissues having a difference can be extracted and displayed.

Further, the comparison of, for example, a normal cell with a disease cell, that of the growth stage or ageing stages of the cells at time series, and that of before medication with after medication or the like can be efficiently executed.

The gene expression information management method further comprises a three-dimensional image creation step of creating a three-dimensional image from two or more pieces of the image data; and an expression level simulation step of simulating expression levels in the three-dimensional image from the expression levels in the image data.

According to this method, a three-dimensional image is created from two or more pieces of the image data, and expression levels in the three-dimensional image are simulated from the expression levels in the image data. Therefore, if slices of an organ are all tested based on one sequence, the three-dimensional image of the organ can be simulated by combining the slice images and the expression level of an mRNA obtained by analyzing each image can be corrected three-dimensionally and displayed.

The gene expression information management method further comprises a typical clone determination step of determining a typical clone from the base sequences belonging to the same cluster, based on at least one of the image data, the base sequences, the expression levels, the information on the extracted tissue, the information on the growth stage or the ageing stage of the extracted tissue, the information as to whether the gene expression is observed, and the information on the region in which the gene expression is observed.

According to this method, a typical clone is determined from the base sequences belonging to the same cluster, based on at least one of the image data, the base sequences, the expression levels, the information on the extracted tissue, the information on the growth stage or the ageing stage of the extracted tissue, the information as to whether the gene expression is observed, and the information on the region in which the gene expression is observed. Therefore, a clone which can be expected to provide the best experimental data can be selected from among the clones derived from the same mRNA and extracted as a typical clone. In addition, EST clones considered to be derived from the same mRNA are classified into the same cluster and an experiment is conducted only to a typical clone in the cluster, whereby the number of experiments can be decreased. In other words, while as many experiments as EST clones have been conventionally required, the sequence clustering enables only the typical clones (as many as the number of clusters) to be experimented.

However, even if the cDNA clones are derived from the same mRNA, sequences thereof slightly differ depending on cut portions. If the in situ hybridization is carried out, some cDNA clones emit fine expression signals and others do not. Due to this, the typical clone may be determined by observing image data and selecting the clone which emits a good expression signal.

Alternatively, the cDNA clone having a base sequence which does not include a genomic repeat sequence or having a sequence length suitable for the experiments may be selected as a typical clone.

The gene expression information management method further comprises a cluster significance determination step of determining a significance of each of the clusters based on at least one of a cluster sequence homology search result, the image data, the base sequences, the expression levels, the information on the extracted tissue, the information on the growth stage or the ageing stage of the extracted tissue, the information as to whether the gene expression is observed, and the information on the region in which the gene expression is observed.

According to this method, the significance of each of the clusters is determined based on at least one of a cluster sequence homology search result, the image data, the base sequences, the expression levels, the information on the extracted tissue, the information on the growth stage or the ageing stage of the extracted tissue, the information as to whether the gene expression is observed, and the information on the region in which the gene expression is observed. Therefore, based on the information, the significance of each cluster can be arbitrarily determined and the cluster which interests the user can be easily discovered.

For example, the significance of a clone which shows a high expression level in a tissue in a specific growth stage or ageing stage can be set high based on information on expression levels and the tissue. Further, If the result of a homology search to the base sequence indicates that there is no hit clone in the existing genetic sequence DB (i.e., a known homologous sequence is not present in the DB), the significance can be set higher.

The gene expression information management method further comprises a genetic locus specification step of specifying a genetic locus on a chromosome in which the base sequences are present; a chromosome map creation step of creating a chromosome map by mapping information on the base sequences on the genetic locus of the chromosome; and a chromosome map display step of displaying the chromosome map created at the chromosome map creation step.

According to this method, it is possible to specify a genetic locus on a chromosome in which the base sequences are present, and create a chromosome map by mapping information (e.g., image data, base sequences, expression levels, information on an extracted tissue, information on the growth stage or ageing stage of the extracted tissue, information as to whether gene expression is observed, and information on a region in which the gene expression is observed) on the base sequences on the genetic locus of the chromosome.

In addition, detailed information on the base sequence may be displayed by selecting a portion of the chromosome map corresponding to the genetic locus (which portion may be indicated by a specific mark).

A computer program according to another aspect of the present invention makes a computer execute an image data input step of inputting pieces of image data on expression of genes; a base sequence input step of inputting base sequences of the expressed genes; a homology search step of conducting a homology search of the sequences input at the base sequence input step, and extracting homologous sequences; and a display step of displaying the image data, the sequences corresponding to the image data, and the homologous sequences.

According to this computer program, image data on the expression of genes is input, base sequences of the expressed genes (e.g., base sequences of cDNA clones) is input, a homology search is conducted to the input sequences to extract homologous sequences, and the image data, the corresponding sequences and the homologous sequences are displayed. Therefore, the genes expressed in the image data can be easily specified.

That is, a homology search (e.g., FastA or Blast) is conducted to known sequences stored in a sequence database (e.g., an EST database or a full-length cDNA database) for the base sequences of cDNA used as probes and corresponding images picked up at a gene expression analysis such as an in situ hybridization experiment, and base sequences having high similarities are displayed. Thus, the cDNA can be specified, and both the cDNA and the image data can be displayed comprehensively.

Further, a similarity (e.g., the score of the homology search) between each base sequence and each of its homologous sequences may be displayed together with the homologous sequence. Thus, the most similar sequence can be displayed. In addition, the homologous sequences can be displayed while being sorted by homology score.

Furthermore, at least one of information attached to each homologous sequence, i.e., the name of a gene, the name of the protein product of the gene, an organism from which the gene is acquired, the name of an organ or a tissue from which the gene is acquired, the ID of the gene in the GenBank, the ID of the protein product of the gene in the GenBank, the length and the similarity by which the base sequence of the cDNA is matched with the sequence of the gene, and information on the evidence of the presence of the gene may be displayed together with the analogous sequence.

In the computer program, at the homology search step, the homology search is conducted for a base sequence of at least one of:
(1) a gene which is known in the same or an other organism;
(2) a gene which is unknown but a cDNA of which is already acquired;
(3) a gene which is unknown but a corresponding genome DNA of which is already acquired;
(4) a gene whose location on a chromosome is known; and
(5) a gene which is already patented.

This illustrates one example of the homology search more concretely. The homology search is conducted for a base sequence of at least one of: (1) a gene which is known to the same or an other organism; (2) a gene which is unknown but a cDNA of which is already acquired; (3) a gene which is unknown but a corresponding genome DNA of which is already acquired; (4) a gene whose location on a chromosome is known; and (5) a gene which is already patented. Therefore, the biological significance of the image data or the like can be easily specified.

That is, since a homology search can be conducted to the respective sequence databases which store base sequences in the categories (1) to (5), the most homologous sequence in each category can be individually specified.

A computer program according to still another aspect of the present invention makes a computer execute an image data input step of inputting pieces of image data on expression of genes; a base sequence input step of inputting base sequences of the expressed genes; a sequence clustering step of sequence clustering the base sequences input at the base sequence input step, and classifying the base sequences into specific clusters; and a display step of displaying the image data and the base sequences corresponding to the image data for each of the clusters.

According to this program, image data on the expression of genes can be input, base sequences of the expressed genes (e.g., base sequences of cDNA clones) can be input, the input base sequences can be clustered to classify the base sequences into specific clusters, and the image data, the corresponding sequences, and the homologous sequences to the corresponding base sequences can be displayed for each cluster. Therefore, by classifying, for example, cDNA (EST sequences) derived from the same mRNA into the same cluster, the base sequences having the same property can be collected and classified into the specific cluster.

This makes the elimination or the like of the duplication of the cDNA clones extracted at random from the cDNA library possible, thereby further improving efficiency for an operation for generalizing experimental results.

The computer program further makes a computer execute a cluster sequencing step of determining a cluster sequence from the base sequences classified into the same cluster at the sequence clustering step, wherein at the display step, the cluster sequence and the base sequences corresponding to the image data are displayed for each of the clusters.

According to this program, a cluster sequence is determined from the base sequences classified into the same cluster, and the cluster sequence, the image data, and the corresponding base sequences are displayed for each cluster. Therefore, a base sequence (e.g., a full-length cDNA) created by combining the base sequences belonging to the same cluster can be determined as the cluster sequence and displayed.

This can facilitate predicting a genetic sequence such as a full-length DNA (mRNA sequence) from an experiment using partial cDNA sequences (EST sequences).

In the computer program, at the sequence clustering step, the base sequences are assembled into a consensus sequence, the base sequences constituting the same consensus sequence are classified into the same cluster, and at the sequence clustering step, the consensus sequence of the cluster is determined as the cluster sequence.

According to this program, the base sequences are assembled into a consensus sequence, the base sequences constituting the same consensus sequence are classified into the same cluster, and the consensus sequence of the cluster is determined as the cluster sequence. Therefore, a cDNA sequence close to a full-length cDNA sequence can be created from partial cDNA sequences using a sequence assembly technique (for creating a long sequence from short sequence fragments. For example, an overlap between sequence fragments is searched by a multiple sequence alignment method or the like, and the sequence fragments having the overlap are synthesized, whereby a longer sequence is created.)

The computer program further makes the computer execute a cluster sequence homology search step of conducting a homology search of the cluster sequence determined at the cluster sequencing step, and extracting homologous sequences, wherein at the display step, the cluster sequence, the homologous sequence to the cluster sequence, and the corresponding sequences are displayed for each of the cluster.

According to this program, a homology search is conducted to the determined cluster sequence to extract homologous sequences, and the cluster sequence, the homologous sequence to the cluster sequence, the image data, and the corresponding base sequences are displays for each of the cluster. This can facilitate specifying the expressed genes in the image data.

That is, a homology search (e.g., FastA or Blast) is conducted to known sequences stored in the sequence database (e.g., the EST database or the full-length cDNA database) for the cluster sequence synthesized from the base sequences of the cDNA clones used as probes by executing a sequence assembly processing or the like, and base sequences having high similarities are displayed. Thus, the expressed genes can be easily specified.

Further, a similarity (e.g., the score of the homology search) between each base sequence and each of its homologous sequences may be displayed together with the homologous sequence. Thus, the most similar sequence to this cluster sequence can be displayed. In addition, the homologous sequences can be displayed while being sorted by homology score.

Furthermore, at least one of information attached to each homologous sequence, i.e., the name of a gene, the name of the protein product of the gene, an organism from which the gene is acquired, the name of an organ or a tissue from which the gene is acquired, the ID of the gene in the GenBank, the ID of the protein product of the gene in the GenBank, the length and the similarity by which the cluster sequence is matched with the sequence of the gene, and information on the evidence of the presence of the gene may be displayed together with the homologous sequence.

Furthermore, if arbitrary genetic sequences are input and clustered with registered cDNA clone sequences, information (e.g., image data) on the cluster sequences belonging to the same cluster can be displayed.

In the computer program, at the cluster sequence homology search step, the homology search is conducts for a base sequence of at least one of:
(1) a gene which is known in the same or an other organism;
(2) a gene which is unknown but a cDNA of which is already acquired;
(3) a gene which is unknown but a corresponding genome DNA of which is already acquired;
(4) a gene whose location on a chromosome is known; and
(5) a gene which is already patented.

This illustrates one example of the homology search more concretely. According to this program, the homology search is conducted for a base sequence of at least one of: (1) a gene which is known to the same or an other organism; (2) a gene which is unknown but a cDNA of which is already acquired; (3) a gene which is unknown but a corresponding genome DNA of which is already acquired; (4) a gene whose location on a chromosome is known; and (5) a gene which is already patented. Therefore, the biological significance of the image data or the like can be easily specified.

That is, since a homology search can be conducted to the respective sequence databases which store base sequences in the categories (1) to (5), the most homologous sequence in each category can be individually specified.

The computer program further makes the computer execute an annotation information storage step of storing at least one of information on an extracted tissue, information on a growth stage or an ageing stage of the extracted tissue, information as to whether gene expression is observed, and information on a region in which the gene expression is observed while making the at least one information correspond to the image data, wherein at the display step, at least one of the information on the extracted tissue, the information on the growth stage or the ageing stage of the extracted tissue, the information as to whether the gene expression is observed, and the information on the region in which the gene expression is observed is displayed while making the at least one information correspond to the image data.

This illustrates one example of the annotation information on the image data more concretely. According to this program, at least one of information on an extracted tissue, information on a growth stage or an ageing stage of the extracted tissue, information as to whether gene expression is observed, and information on a region in which the gene expression is observed can be stored while making the at least one information correspond to the image data, and at least one of the information on the extracted tissue, the information on the growth stage or the ageing stage of the extracted tissue, the information as to whether the gene expression is observed, and the information on the region in which the gene expression is observed can be displayed while making the at least one information correspond to the image data.

The computer program further makes the computer execute an expression level estimation step of estimating expression levels of the genes in the image data based on one of or both of the image data and the base sequences.

According to this computer program, expression levels of the genes in the image data are estimated based on one of or both of the image data and the base sequences. This can facilitate specifying an expression pattern (a pattern of uniform expression, non-uniform expression or the like).

The expression levels may be estimated by obtaining the signal intensity and the area of signal region of a fluorescent dye or the like in the image data by means of a known image analysis method or the like. Further, by using information on not only the image data but also the base sequences, an automatic estimation can be made as follows. If a genomic repeat sequence, for example, is included in the base sequences, the probability of cross-hybridization (occurrence of a hybridization reaction to other mRNA having the same genomic repeat sequence) is high. Therefore, the reliability of the estimated expression level is low.

Moreover, if images of the same tissue section in which the probes are made illuminant and in which the probes are not made illuminant are picked up or images of the same tissue section which are colored with different fluorescent dyes are picked up, the image analysis processing can be easily executed (by, for example, estimating an expression level by obtaining the difference between the two images).

The computer program further makes the computer execute an expression level order sorting step of sorting display orders of the image data according to the expression levels estimated at the expression level estimation step.

Since it is possible to sort display orders of the image data according to the estimated expression levels according to this program, the user can efficiently check the experimental result.

The computer program according further makes the computer execute an image comparison step of comparing two or more pieces of the image data based on at least one of the image data, the base sequences, the expression levels, the information on the extracted tissue, the information on the growth stage or the ageing stage of the extracted tissue, the information as to whether the gene expression is observed, and the information on the region in which the gene expression is observed; and a difference extraction step of extracting a difference among the two or more pieces of the image data based on a comparison result of the image comparison step.

According to this computer program, two or more pieces of the image data are compared based on at least one of the image data, the base sequences, the expression levels, the information on the extracted tissue, the information on the growth stage or the ageing stage of the extracted tissue, the information as to whether the gene expression is observed, and the information on the region in which the gene expression is observed, and a difference among the two or more pieces of the image data is extracted based on a comparison result. Therefore, it is possible to efficiently extract the difference among the images.

Thus, if annotation processings are carried out for the expression patterns in the respective tissues by image recognition or manual operation, and annotation results are automatically compared, the images of the tissues having a difference can be extracted and displayed.

Further, the comparison of, for example, a normal cell with a disease cell, that of the growth stage or ageing stages of the cells at time series, and that of before medication with after medication or the like can be efficiently executed.

The computer program further makes the computer execute a three-dimensional image creation step of creating a three-dimensional image from two or more pieces of the image data; and an expression level simulation step of simulating expression levels in the three-dimensional image from the expression levels in the image data.

According to this computer program, a three-dimensional image is created from two or more pieces of the image data, and expression levels in the three-dimensional image are simulated from the expression levels in the image data. Therefore, if slices of an organ are all tested based on one sequence, the three-dimensional image of the organ can be simulated by combining the slice images and the expression level of an mRNA obtained by analyzing each image can be corrected three-dimensionally and displayed.

The computer program further makes the computer execute a typical clone determination step of determining a typical clone from the base sequences belonging to the same cluster, based on at least one of the image data, the base sequences, the expression levels, the information on the extracted tissue, the information on the growth stage or the ageing stage of the extracted tissue, the information as to whether the gene expression is observed, and the information on the region in which the gene expression is observed.

According to this computer program, a typical clone is determined from the base sequences belonging to the same cluster, based on at least one of the image data, the base sequences, the expression levels, the information on the extracted tissue, the information on the growth stage or the ageing stage of the extracted tissue, the information as to whether the gene expression is observed, and the information on the region in which the gene expression is observed. Therefore, a clone which can be expected to provide the best experimental data can be selected from among the clones derived from the same mRNA and extracted as a typical clone. In addition, EST clones considered to be derived from the same mRNA are classified into the same cluster and an experiment is conducted only to a typical clone in the cluster, whereby the number of experiments can be decreased. In other words, while as many experiments as EST clones have been conventionally required, the sequence clustering enables only the typical clones (as many as the number of clusters) to be experimented.

However, even if the cDNA clones are derived from the same mRNA, sequences thereof slightly differ depending on cut portions. If the in situ hybridization is carried out, some cDNA clones emit fine expression signals and others do not. Due to this, the typical clone may be determined by observing image data and selecting the clone which emits a good expression signal.

Alternatively, the cDNA clone having a base sequence which does not include a genomic repeat sequence or having a sequence length suitable for the experiments may be selected as a typical clone.

The computer program further making the computer execute a cluster significance determination step of determining a significance of each of the clusters based on at least one of a cluster sequence homology search result, the image data, the base sequences, the expression levels, the information on the extracted tissue, the information on the growth stage or the ageing stage of the extracted tissue, the information as to whether the gene expression is observed, and the information on the region in which the gene expression is observed.

According to this computer program, the significance of each of the clusters is determined based on at least one of a cluster sequence homology search result, the image data, the base sequences, the expression levels, the information on the extracted tissue, the information on the growth stage or the ageing stage of the extracted tissue, the information as to whether the gene expression is observed, and the information on the region in which the gene expression is observed. Therefore, based on the information, the significance of each cluster can be arbitrarily determined and the cluster which interests the user can be easily discovered.

For example, the significance of a clone which shows a high expression level in a tissue in a specific growth stage or ageing stage can be set high based on information on expression levels and the tissue. Further, If the result of a homology search to the base sequence indicates that there is no hit clone in the existing genetic sequence DB (i.e., a known homologous sequence is not present in the DB), the significance can be set higher.

The computer program further makes the computer execute a genetic locus specification step of specifying a genetic locus on a chromosome in which the base sequences are present; a chromosome map creation step of creating a chromosome map by mapping information on the base sequences on the genetic locus of the chromosome; and a chromosome map display step of displaying the chromosome map created at the chromosome map creation step.

According to this computer program, it is possible to specify a genetic locus on a chromosome in which the base sequences are present, and create a chromosome map by mapping information (e.g., image data, base sequences, expression levels, information on an extracted tissue, information on the growth stage or ageing stage of the extracted tissue, information as to whether gene expression is observed, and information on a region in which the gene expression is observed) on the base sequences on the genetic locus of the chromosome.

In addition, detailed information on the base sequence may be displayed by selecting a portion of the chromosome map corresponding to the genetic locus (which portion may be indicated by a specific mark).

In addition, the present invention relates to a recording medium. The recording medium according to the present invention records the above program.

According to this recording medium, the program can be realized using a computer and the same advantages as those of the respective methods executed by the program can be attained by allowing the computer to read the program recorded on the recording medium and to execute the program.

An in situ hybridization analysis management method according to the present invention comprises a master library production step of producing a master library of genetic clones, and aligning the genetic clones on a multiwell plate; a sequencing step of reading nucleotide information on the genetic clones produced at the master library production step; a sequence analysis step of performing an analysis based on the nucleotide sequence information read at the sequencing step; a hybridization step of conducting an in situ hybridization experiment using the genetic clones produced at the master library step, and one of a specific cell, a specific tissue, and a specific organ; and a progress management step of managing a progress of steps other than the progress management step according to an analysis result of the sequence analysis step.

According to this method, a master library of genetic clones is produced to align the genetic clones aligned on a multiwell plate, nucleotide information on the genetic clones produced is read, an analysis is performed based on the nucleotide sequence information read, an in situ hybridization experiment is conducted using the genetic clones produced, and one of a specific cell, a specific tissue, and a specific organ, and a progress of the other steps is managed according to an analysis result. Therefore, it is possible to generally manage the in situ hybridization experiment, efficiently execute the respective steps, and avoid doing over the experiment again or conducting an unnecessary experiment.

In addition, if a normal tissue and a disease tissue, for example, are acquired and the large-scale in situ hybridization analysis management method according to the present invention is applied, disease related genes for a drug design can be efficiently, accurately searched, as compared with the conventional analysis method or the like using DNA chips or the like.

Furthermore, if tissues to which a poison and a drug have been applied, for example, are acquired and the large-scale in situ hybridization analysis management method according to the present invention is applied, a movement search and a toxic search based on the detection of a change in a gene expression pattern can be efficiently, accurately conducted, as compared with the conventional analysis method or the like using DNA chips or the like.

Besides, if the large-scale in situ hybridization analysis management method according to the present invention is applied, the estimation of the correlation between genes (proteins) similar in expression pattern or equal in localization and the network search between the genes (proteins) can be efficiently, accurately conducted. In addition, the functions of the respective genes can be efficiently, accurately estimated, as compared with the conventional method.

If an early embryo is acquired and the large-scale in situ hybridization analysis management method according to the present invention is applied, gene search for regenerative medicine such as gene search for differentiation can be efficiently, accurately conducted.

Moreover, if information on a gene and the like is acquired through a network such as the Internet and the large-scale in situ hybridization analysis management method according to the present invention is applied, information on the expression pattern of the gene in a specific tissue can be sent back through the network.

Further, the present invention relates to an in situ hybridization analysis management apparatus. The in situ hybridization analysis management apparatus according to the present invention comprises a master library plate information management unit which manages master library plate information on master library plates used in an in situ hybridization experiment; a master library plate information output unit which outputs the master library plate information managed by the master library plate information management unit; a sequence analysis unit which acquires base sequence data on genetic clones output from a DNA sequencer, which conducts a sequence cleaning to the base sequence data, which identifies genes, and which executes sequence clustering the identified genes; a sequence and expression image data management unit which acquires the sequence data together with data on expression images from the in situ hybridization experiment on one of a specific cell, a specific tissue, and a specific organ, and which manages the base sequence data and the expression image data while making the base sequence data and the expression image data correspond to each other; and an analysis management unit which manages at least one of the master library plate information management unit, the mater library plate information output unit, the sequence analysis unit, and the sequence and expression image data management unit.

This apparatus manages master library plate information on master library plates used in an in situ hybridization experiment, outputs the master library plate information managed, acquires sequence data on genetic clones output from a DNA sequencer, conducts a sequence cleaning to the sequence data, identifies genes, executes sequence clustering the identified genes, acquires the sequence data together with data on expression images from the in situ hybridization experiment on one of a specific cell, a specific tissue, and a specific organ, manages the sequence data and the expression image data while making the sequence data and the expression image data correspond to each other, manages the progress of at least one of the master library plate information management, the master library plate information output, the sequence analysis, and the sequence and expression image data management. Therefore, it is possible to generally manage the in situ hybridization experiment, efficiently execute the respective steps, and avoid doing over the experiment again or conducting an unnecessary experiment.

An in situ hybridization apparatus according to still another aspect of the present invention comprises a unit which displays a plate information management screen on a monitor for a user so that the user inputs and checks information on produced master library plates through an input device, which registers the information input by the user in a predetermined region of a master library database, and which stores an analysis progress status of each of the master library plates as "sequencing step unfinished"; a unit which extracts the information on the plates each having the analysis progress status of "sequencing step unfinished" based on the information on the master library plates newly registered in the master library database, which displays an analysis progress status management screen on the monitor, and which thereby notifies the user of the extracted plate information; a unit which acquires sequence data on the plates designated by sequencing step unfinished plate information, the sequence data output from a DNA sequencer, which stores the acquired sequence data in a predetermined storage region of a sequence database, which updates the analysis progress status of each of the designated plates to "sequence analysis step unfinished", and which displays a "date" in a sequencing space and "standby for analysis" in a sequence analysis pace of each of the plates the sequence data of which is obtained on the analysis progress status management screen; a unit which notifies a sequence analysis section of the plates each having the analysis progress status of "sequence analysis step unfinished", which updates the analysis progress status of each of the plates to "now being sequence-analyzed", which displays "analysis in progress" in the sequence analysis space of the each plate on the analysis progress status management screen, and which updates display of a status space of the each plate from "standby for sequence analysis" to "now being sequence-analyzed"; a unit which causes the sequence analysis section to acquire the sequence data on the plates each having the analysis progress status of "sequence analysis step unfinished" from the sequence database, to execute a sequence analysis processing, to register an execution result in an analysis result database, and to notify a plate management section of quality information on the sequence data, the quality information being made clear as a result of the analysis; a unit which updates the analysis progress status of each of the plates in the master library database from "now being sequence-analyzed" to "standby for hybridization", which displays on the analysis progress status management screen the date in the sequence analysis space of each of the plates completed with the sequence analysis, which displays on the analysis progress status management screen the quality information obtained as a result of the sequence analysis in a quality space, and which updates the display of the status space of the each plate from "now being sequence-analyzed" to "standby for hybridization"; a unit which causes the plate management section to output, to the analysis progress status management screen, information on the plates an NG clone rate of each of which satisfies a standard among the plates each having the analysis progress status of "sequence analysis step finished" "in situ hybridization step unfinished"; and unit which displays "standby for analysis" in a hybridization space of the plates the NG clone rate of each of which satisfies the standard on the analysis progress status management screen, and which displays "terminate analysis" and "terminate analysis" in the hybridization space and the status space of each of the plates the NG clone rate of which does not satisfy the standard, respectively, and which updates the analysis progress status of each of the plates the NG clone rate of which does not satisfy the standard from "standby for hybridization" to "terminate analysis".

This apparatus manages the progress of the master library plate information, the master library plate information output, the sequence analysis, and the sequence and expression image data in the in situ hybridization experiment. Therefore, it is possible to generally manage the in situ hybridization experiment, efficiently execute the respective steps, and avoid doing over the experiment or conducting an unnecessary experiment.

Namely, this apparatus produces a master library database, manages information on the produced master library database, and manages the analysis progress status of each plate on the master library database by one of "sequencing step unfinished", "sequence analysis unfinished", "now being sequence-analyzed", "standby for hybridization", "standby for analysis", "terminate analysis". Therefore, the analysis progress statuses of the plates can be managed unitarily.

Moreover, by displaying the analysis progress status management screen on the monitor, this apparatus can notify the user of the analysis progress status of each plate and the content of the experiment in detail.

The other objects, features and advantages of the present invention are specifically set forth in or will become apparent from the following detailed descriptions of the invention when read in conjunction with the accompanying drawings.

### BRIEF DESCRIPTION OF THE DRAWINGS

Fig. 1 is a block diagram illustrating the basic system configuration of the present invention;
Fig. 2 is a principle block diagram illustrating the basic principle of the present invention;
Fig. 3 is a flow chart illustrating one example of the synchronous processing of a system in one embodiment according to the present invention;
Fig. 4 is a block diagram illustrating one example of the configuration of the system to which the present invention is applied;
Fig. 5 is a flow chart illustrating one example of the image annotation information input processing of the system in this embodiment;
Fig. 6 is a flow chart illustrating one example of the cDNA clone sequence homology search processing of the system in this embodiment;
Fig. 7 is a flow chart illustrating one example of the sequence assembly processing of the system in this embodiment;
Fig. 8 is a flow chart illustrating one example of the cluster sequence homology search processing of the system in this embodiment;
Fig. 9 is a flow chart illustrating one example of the three-dimensional simulation processing of the system in this embodiment;
Fig. 10 is a flow chart illustrating one example of the expression level estimation processing of the system in this embodiment;
Fig. 11 is a flow chart illustrating one example of the image comparison processing of the system in this embodiment;
Fig. 12 is a flow chart illustrating one example of the chromosome map creation processing of the system in this embodiment;
Fig. 13 is an illustration of one example of an annotation information input screen displayed on a monitor;
Fig. 14 is an illustration of one example of a list report screen displayed if data on each cDNA clone is to be viewed;
Fig. 15 is an illustration of one example of a detailed report screen displayed if data on each cDNA clone is to be viewed;
Fig. 16 is an illustration of another example of the detailed report screen displayed if data on each cDNA clone is to be viewed;
Fig. 17 is an illustration of one example of a list report screen displayed if data on each cluster is to be viewed;
Fig. 18 is an illustration of one example of a detailed report screen if data on each cluster is to be viewed;
Fig. 19 is an illustration of one example of a chromosome map display screen displayed if the chromosome map is to be viewed;
Fig. 20 is an illustration for explaining one example of a master library plate and derivative plates thereof;
Fig. 21 is an illustration for explaining the advantages of the present invention;
Fig. 22 is illustrations for explaining the advantages of the present invention;
Fig. 23 is an illustration of the outline of a large-scale in situ hybridization method;
Fig. 24 is an illustration of one example of a photographed image;
Fig. 25 is an illustration of one example of a plate information management screen displayed on the monitor;
Fig. 26 is an illustration of one example of an analysis progress status management screen displayed on the monitor;
Fig. 27 is an illustration of one example of the analysis progress status management screen displayed on the monitor;
Fig. 28 is an illustration of one example of the analysis progress status management screen displayed on the monitor;
Fig. 29 is an illustration of one example of the analysis progress status management screen displayed on the monitor;
Fig. 30 is an illustration of one example of the analysis progress status management screen displayed on the monitor;
Fig. 31 is an illustration of one example of the analysis progress status management screen displayed on the monitor;
Fig. 32 is an illustration of one example of the analysis progress status management screen displayed on the monitor;
Fig. 33 is an illustration of one example of the analysis progress status management screen displayed on the monitor; and
Fig. 34 is a principle block diagram illustrating the basic principle of the present invention.

### BEST MODE FOR CARRYING OUT THE INVENTION

Exemplary embodiments of a gene expression information management system, a gene expression information management method, a program, an in situ hybridization analysis management method, and an in situ hybridization analysis management system will be explained hereinafter in detail with reference to the drawings. It should be noted that the present invention is not limited by these embodiments.

In the embodiments, an example of applying the present invention to the collection of experimental data by the large-scale in situ hybridization will be explained. However, the present invention can be applied not only to this example but also to other examples of using the other experimental methods for analyzing gene expression information by other in situ hybridization methods.

### [The outline of the present invention]

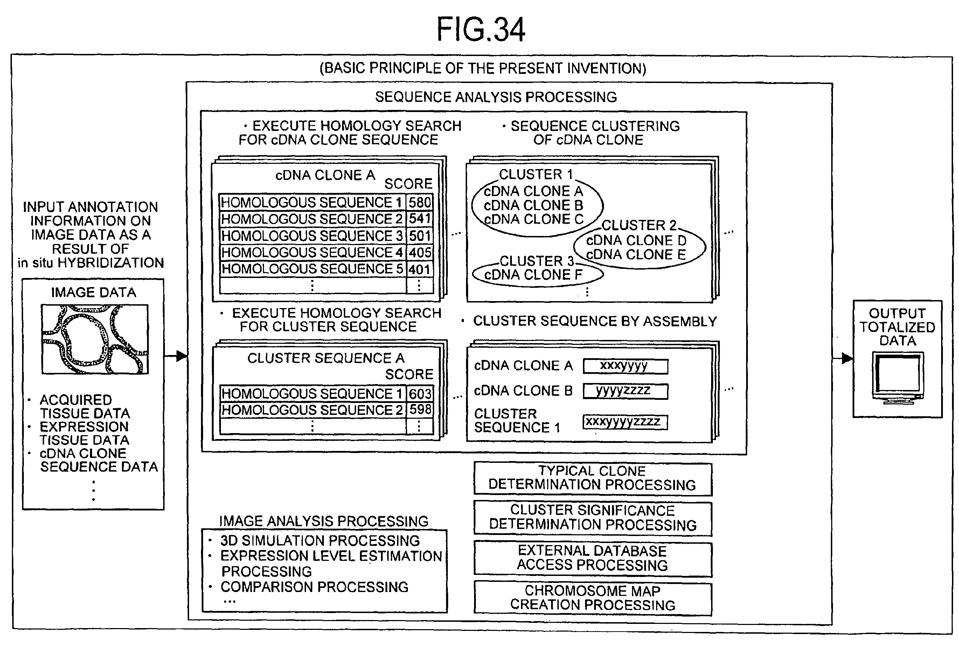

The outline of the present invention will be explained first, and the configuration, the processings, and the like of the present invention will then be explained in detail. Fig. 34 is a principle block diagram illustrating the basic principle of the present invention.

The present invention has roughly the following basic features. As shown in Fig. 34, the present invention stores base sequence data on an expressed gene (cDNA) corresponding to image data on an in situ hybridization result, and makes a user input annotation information (information on an extracted tissue, information on the growth stage or ageing stage of the extracted tissue, information on whether a gene expression is observed, information on such region as cells in which the gene expression is observed). In addition, the present invention automatically recognizes these pieces of annotation information from the image data using a known image analysis technique.

The present invention conducts a homology search to known base sequences stored in a sequence database (e.g., the EST database or a full-length cDNA database) for the input base sequence of the cDNA, extracts homologous sequences to the input base sequence, and displays a base sequence corresponding to the image data, homologous sequences to the displayed base sequence, homology scores, and the like.

Further, the present invention conducts a homology search to the base sequence of at least one of a gene which is already known to the same or different organism, a gene which is unknown but the cDNA of which is already acquired, a gene which is unknown but a genome DNA section corresponding to which is already acquired, a gene the location of which on a chromosome is known, and a gene which is already patented.

The present invention collects base sequences having the same property and classifies the collected base sequences into specific clusters by, for example, classifying cDNA clones (EST sequences) derived from the same mRNA into the same cluster. In addition, the present invention determines cluster sequences from the base sequences classified into the same cluster, and displays a cluster sequence and a base sequence corresponding to the image data for each cluster. Further, the present invention may assemble consensus sequences using the base sequences, classify the base sequences that constitute the same consensus sequence into the same class, and determine the consensus sequence of each cluster as a cluster sequence.

The present invention conducts a homology search to the determined cluster sequences to extract homologous sequences, and displays a cluster sequence, homologous sequences to the cluster sequences, and the base sequence corresponding to the image data for each cluster.

The present invention estimates the expression level of a gene in the image data based on one of or both of the image data and the base sequences. Further, the present invention may sort display orders of the image data according to the estimated expression levels.

Further, the present invention compares two or more pieces of image data based on at least one of information on the image data, base sequences, expression level, and the extracted tissue, information on the growth stage or ageing stage of the extracted tissue, information as to whether gene expression is observed, and information on regions in which the gene expression is observed, and extracts differences among the two or more pieces of image data based on the comparison result.

The present invention creates a three-dimensional image from the two or more pieces of image data, and simulates the expression level of the three-dimensional image from that of the image data.

The present invention determines a typical clone from the base sequences belonging to the same cluster based on at least one of the information on the image data, the information on the extracted tissue, the information on the growth stage or ageing stage of the extracted tissue, the information as to whether gene expression is observed, and the information on regions in which the gene expression is observed.

The present invention determines cluster significance based on at least one of the cluster sequence homology search result, the information on the image data, the information on the extracted tissue, the information on the growth stage or ageing stage of the extracted tissue, the information as to whether gene expression is observed, and the information on regions in which the gene expression is observed.

Furthermore, the present invention creates a chromosome map by identifying a genetic locus on the chromosome in which a base sequence is present, and mapping information on the base sequence (e.g., the image data, the base sequences, the expression levels, and the extracted tissue, the information on the growth stage or ageing stage of the extracted tissue, the information as to whether gene expression is observed, and the information on regions in which the gene expression is observed) on the genetic locus of the chromosome.

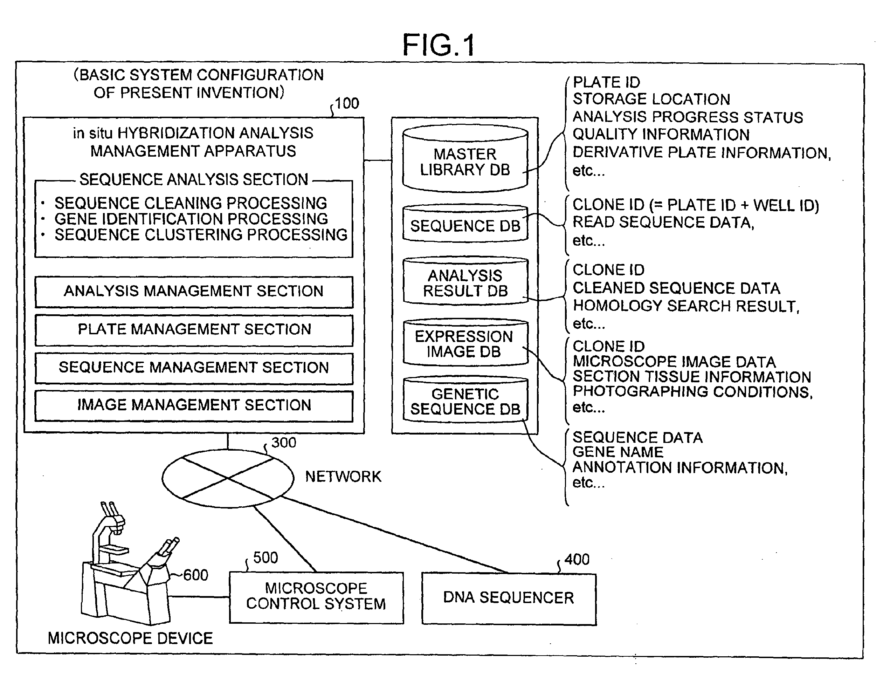

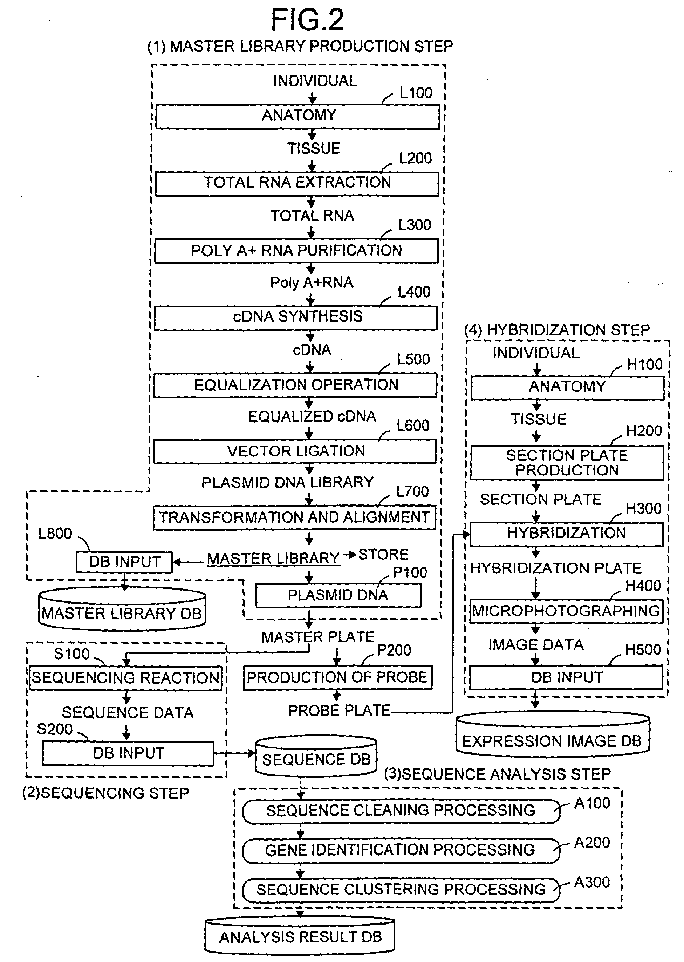

The other outline of the present invention will be explained. Fig. 1 is a block diagram illustrating the basic system configuration of the present invention. Fig. 2 is a principle block diagram illustrating the basic principle of the present invention. As shown in Fig. 1, a large-scale in situ hybridization analysis management system which realizes a large scale in situ hybridization analysis management method according to the present invention is roughly constituted so that an in situ hybridization analysis management apparatus 100, a DNA sequencer 400, and a microscope control system 500 are connected to be communicable to one another through a network 300.

The present invention roughly has the following basic features. As shown in Fig. 2, the present invention is a large-scale in situ hybridization analysis management method including: (1) a master library production step; (2) a sequencing step; (3) a sequence analysis step; and (4) a hybridization step.

The respective steps of the large-scale in situ hybridization analysis management method executed by the large-scale in situ hybridization analysis management system according to the present invention will be explained in this order.

### (1) Master library production step

At this step, genetic clones aligned on a multiwell plate are produced. An ordinary genetic engineering technique for producing a collection of genes expressed in a specific tissue is employed. The master library production step includes the following steps L100 to L800 and P100 to P200.

### (Step L100: Anatomy)

An individual is anatomized to take out a specific tissue or organ.

### (Step L200: Total RNA extraction)

Using an ordinary experimental technique, Total RNA is extracted from the tissue or organ obtained at the step L100.

### (Step L300: Poly A+ RNA purification)

Using an ordinary experimental technique, mRNA are selectively extracted from the Total RNA obtained at the step L200.

### (Step L400: cDNA synthesis)

Using an ordinary experimental technique, cDNA sequences are synthesized from the mRNA's obtained at the step L300 using reverse transcriptase.

### (Step L500: Equalization operation)

Using an ordinary experimental technique, quantity ratios of the cDNA obtained at the step L400 are equalized. This technique is intended to prevent the duplication of a master library obtained finally. It should be noted that, the step L500 may be omitted.

### (Step L600: Vector ligation)

Using an ordinary experimental technique, the cDNA obtained at the step L500 are integrated into a vector.

### (Step L700: Transformation and alignment)

Using an ordinary experimental technique, the cDNA obtained at the step L600 are isolated and aligned on a multiwell plate. The plate produced at this master library production step is stored as a master library in a freezer.

### (Step L800: DB input)

Information on a plate ID, a storage location and the like of the master library plate obtained at the step L700 is input in a database (hereinafter "DB"). Namely, the in situ hybridization analysis management apparatus 100 makes an operator input information on the plate on a plate information management screen to be explained later, and stores the input information in the master library DB.

### (Step P100: Plasmid DNA)

As a preprocessing for use in later operations, plasmid vectors with which cDNA are integrated are extracted from the master library produced at the step L700 using an ordinary experimental technique.

### (2) Sequencing step

At this step, nucleotide sequences of the genetic clones produced at the master library production step are read using an ordinary experimental technique. For example, using a commercially available DNA sequencing machine such as ABI3700 (product name) manufactured by Applied Biosystems (company name), the nucleotide sequences of the genetic clones can be read. This sequencing step includes the following steps S100 to S200.

### (Step S100: Sequencing reaction)

Using an ordinary experimental technique, the DNA sequencer 400 reads the plasmid DNA sequences obtained at the step P100.

### (Step S200: DB input)

The sequences read at the step S100 are input to a DB. Namely, the in situ hybridization analysis management apparatus 100 acquires the sequence data read by the DNA sequencer 400, and stores the acquired data in a sequence DB.

### (3) Sequence analysis step

Analysis is performed based on the nucleotide sequence information read at the sequencing step. For example, an ordinary EST analysis program can be utilized for the analysis. Through this step, information on the qualities of the genetic clones and corresponding genes can be obtained. This sequence analysis step includes the following steps S100 to A300. In addition, the in situ hybridization analysis management apparatus 100 outputs information on the plate which is not subjected yet to the sequencing step onto an analysis progress status management screen to be explained later, and allows the operator to check the information on the plate which is not subjected yet to the sequencing step. The operator can issue an instruction to start the following sequence analysis step to the in situ hybridization analysis management apparatus 100 for the plate designated by the information on the plate which is not subjected yet to the sequencing step according to a predetermined operation.

### (Step A100: Sequence cleaning processing)

The in situ hybridization analysis management apparatus 100 subjects the sequence information read by the DNA sequencer 400 to quality trimming by the processing of a sequence analysis section. At this time, a vector sequence part is also trimmed. This step A100 can be realized by, for example, an existing software (e.g., phred or cross_match developed by University of Washington). The in situ hybridization analysis management apparatus 100 determines the result of this step by the processing of the sequence analysis section, to enable only the sequences having sufficient lengths and qualities to be analyzed later.

### (Step A200: Gene identification processing)

The in situ hybridization analysis management apparatus 100 determines on which gene the sequence information obtained at the step S100 is. This step S200 can be realized by conducting a homology search (e.g., blast of National Center for Biotechnology Information (hereinafter, "NCBI")) to the sequence data stored in a genetic sequence DB such as GenBank.

### (Step A300: Sequence clustering processing)

The in situ hybridization analysis management apparatus 100 clusters the entire collection of sequences obtained at the step S100 according to the similarities of sequences to thereby exclude duplication. This step S300 can be realized by an existing software such as blastclust developed by NCBI. As a result of this step, the number of gene species included in the genetic clone collection can be estimated. Further, by unifying the clones derived from the same genes, the number of times of hybridization can be decreased.

The steps A200 and A300 can be executed in an ordinary order.

The in situ hybridization analysis management apparatus 100 displays quality information on each plate completed with the sequence analysis step on the analysis progress status management screen to be explained later. Thus, information used as a standard of judgment as to whether the operator conducts the hybridization step can be displayed. The in situ hybridization analysis management apparatus 100 outputs hybridization step unfinished plate information on the analysis progress status management screen for the plate the quality of which is kept to some extent so that the operator can check the hybridization step unfinished plate information.

### (4) Hybridization step

An in situ hybridization experiment is conducted. As a result of the in situ hybridization experiment conducted at this hybridization step, the expression statuses of genes corresponding to the genetic clones in the specific cell, tissue or organ can be recognized. This step includes the following steps H100 to H500.

### (Step H100: Anatomy)

An individual is anatomized to take out a specific tissue or organ.

### (Step H200: Section plate production)

At this step, an operation for fixing sections sliced from the tissue obtained at the step H100 onto the multiwell plate is carried out.

### (Step P200: Probe production)

Using an ordinary experimental technique, cDNA sequence parts are transcribed from the plasmid DNA's obtained at the step P100 to thereby produce probes.

### (Step H300: Hybridization)

Hybridization is conducted by an arbitrary combination of the probe obtained at the step P200 and the section obtained at the step H200. This step is an ordinary technique for checking expression information on a gene corresponding to the probe on a section tissue.

### (Step H400: Microphotographing)

A microscope control system 500 microphotographs each well of the hybrid-plate (multiwell plate for which hybridization is conducted).

### (Step H500: DB input)

Microscopic image data obtained at the step H400 is input into an image DB. Namely, the in situ hybridization analysis management apparatus 100 acquires data on the expression images photographed at the step H400 from the microscope control system 500, and stores the acquired data in an expression image DB. The in situ hybridization analysis management apparatus 100 performs various analysis processings based on the content of the sequence DB produced at the step S200 and that of the expression image DB produced at this step, and stores analysis results in an analysis result DB.

Referring back to Fig. 1, the large-scale in situ hybridization analysis management system which realizes the large-scale in situ hybridization analysis management method according to the present invention will be described in detail.

As shown in Fig. 1, the large-scale in situ hybridization analysis management system which realizes the large-scale in situ hybridization analysis management method is roughly constituted so that the in situ hybridization analysis management apparatus 100, the DNA sequencer 400, and the microscope control system 500 are connected to be communicable to one another through the network 300.

As shown in Fig. 1, the in situ hybridization analysis management apparatus 100 roughly consists of a sequence analysis section, an analysis management section, a plate management section, a sequence management section, and an image management section. In addition, the in situ hybridization analysis management apparatus 100 is accessible to the master library DB, the sequence DB, the analysis result DB, the expression image DB, and the genetic sequence DB.

In the master library DB shown in Fig. 1 information such as an ID of each plate, a storage location thereof, an analysis progress status thereof, quality information thereon, the presence/absence of derivative plates thereof, and storage locations of the respective derivative plates while associating them with one another.

The sequence DB stores an ID of each clone (= a plate ID + a well ID), read sequence data (chromatogram data, nucleotide sequence data) on the clone, and the like.

The analysis result DB stores an ID of each clone, cleaned sequence data thereon, a homology search result therefor, and the like.

The expression image DB stores an ID of each clone, microscopic image data, information on a photographed section tissue, various photographing conditions, and the like.

The sequence analysis section serves as a sequence analysis unit which acquires base sequence data on the genetic clones output from the DNA sequencer, cleans the sequences for the base sequence data, identifies genes, and clusters the identified genes. The sequence analysis section executes (1) a sequence cleaning processing, (2) a gene identification processing, and (3) a sequence clustering processing. These processings will now be explained.

### (1) Sequence cleaning processing

Quality trimming and vector trimming are carried out based on determined sequence data. Based on the output data (chromatogram data) of the DNA sequencer, the quality trimming and the vector trimming can be realized by, for example, phred and cross_match programs developed by University of Washington, respectively. As a result of trimming, it is ensured that the insert sequence of each clone has a sufficient length and a sufficient quality. Namely, the result of the sequence cleaning processing can be also used as quality data on each clone. "Quality trimming" means herein a processing for clipping both ends of each read sequence that are low in quality. "Vector trimming" means herein a processing for clipping sequences of cloning vector parts among the read sequences. As a result of these processings, only base sequence information on the insert sequence is extracted.

### (2) Gene identification processing

Based on the base sequence information on the insert sequence part obtained as a result of the sequence cleaning processing, a homology search is conducted to the genetic sequence DB to thereby identify genes. This processing can be realized by using, for example, the blast program developed by NCBI or the like.

### (3) Sequence clustering processing

Duplicated sequences are removed from the collection of the base sequence information on the insert sequence part obtained as a result of the sequence cleaning processing based on sequence similarity. As a result of the sequence clustering processing, duplicated clones are detected and the number of genetic species is estimated. This processing can be realized by, for example, the blastclust program developed by NCBI or the like.

The plate management section shown in Fig. 1 serves as a master library plate information management unit which manage master library plate information on the master library plate employed in the in situ hybridization experiment, and a master library plate information output unit which outputs master library plate information managed by the master library plate information management unit.

The sequence management section and the image management section shown in Fig. 1 serve as a sequence/expression image data management unit which acquires expression image data photographed at the in situ hybridization experiment using the genetic clones of the base sequence data and the specific cell, tissue or organ, and which manages the base sequence data and the expression image data while making them correspond to each other.

The analysis management section shown in Fig. 1 serves as an analysis management unit which manages the progress of at least one of the master library plate information management unit, the master library plate information output unit, the sequence analysis unit, and the sequence/expression image data management unit.

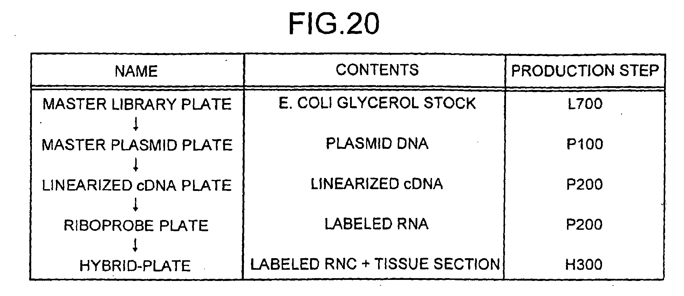

The master library plate and derivative plates from the master library plate used in the present invention will next be explained with reference to Fig. 20.

As described above, the large-scale in situ hybridization analysis management system produces various plates from the master library plate in an experimental process, and finally completes the microphotographing of the hybrid-plate, thus finishing a series of analysis steps.

The finally obtained images represent the expression states of the genes corresponding to the genetic clones stored in the wells of the master library plate.

According to the present invention, the plates which are produced as intermediates (derivative plates) since the production of the master library plate until that of the hybrid-plate are temporarily stored so that analysis steps can be done over again halfway along the steps. Fig. 20 illustrates such derivative plates. Namely, Fig. 20 is an illustration for explaining one example of the master library plate used in the present invention as well as the derivative plates from the master library plate. The plates shown in Fig. 20 are given only for illustrative purposes and not always essential to the system according to the present invention. In Fig. 20, each arrow represents a derivative relationship. In other words, a plate at the end point of the arrow is produced based on a plate at the start point of the arrow. The arrow normally represents a one-to-many correspondence. For example, a master plasmid plate can be produced a plurality of number of times using one master library plate.

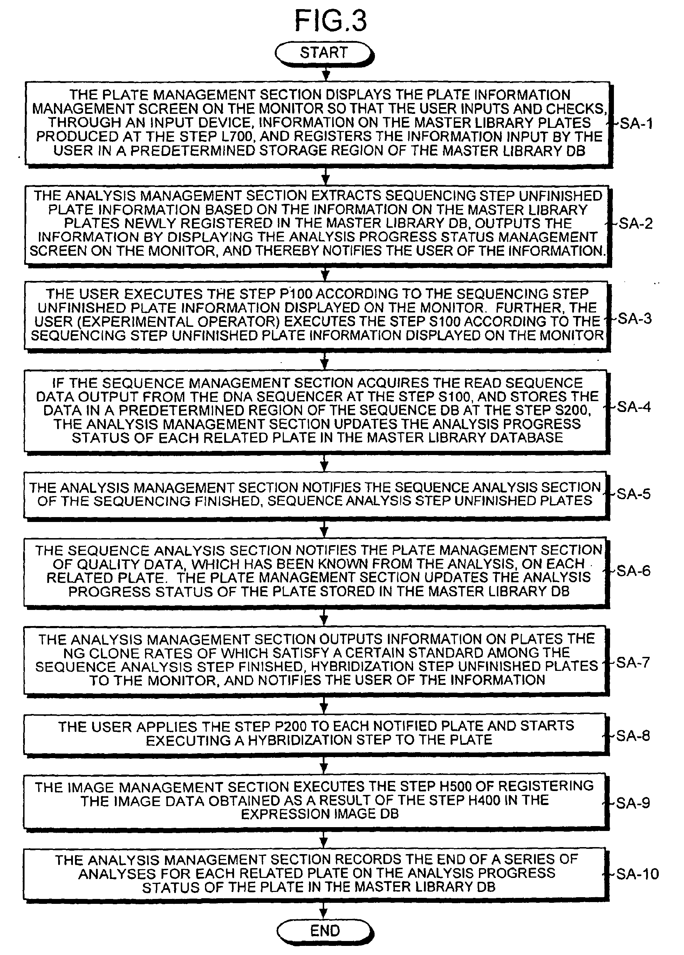

A processing flow (synchronous mechanism) from the registration of the master library to analysis in the large-scale in situ hybridization analysis management system according to the present invention will next be explained with reference to Fig. 3. Fig. 3 is a flow chart illustrating one example of the synchronous processing of the system in this embodiment.

The plate management section according to the present invention displays the plate information management screen on the monitor so that the user inputs and checks, through an input device, information on the master library plates produced at the step L700 (e.g., each plate ID, a storage location of each plate, an analysis progress status thereof, quality information thereon, presence/absence of derivative plates, and storage locations of the respective derivative plates), and registers the information input by the user in a predetermined storage region of the master library DB (at a step SA-1).

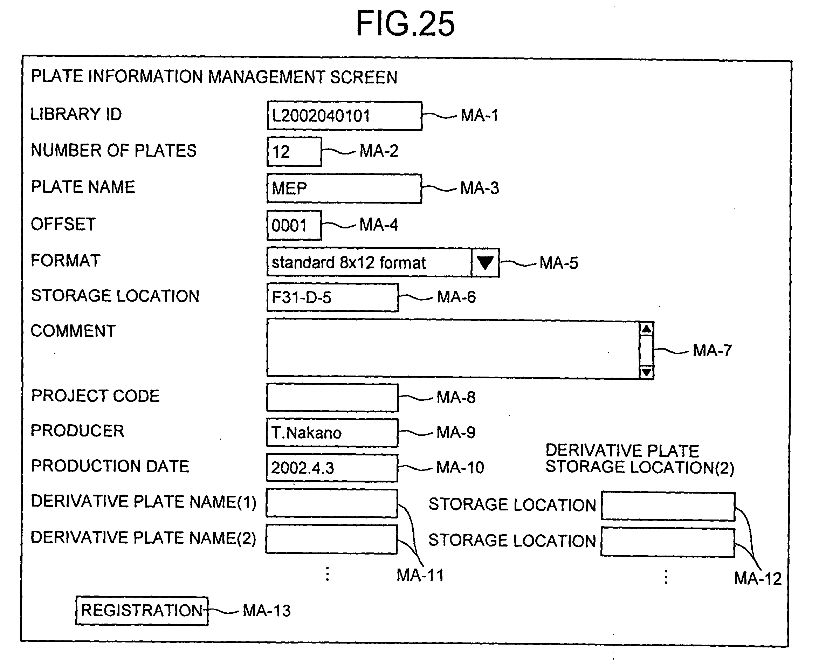

Fig. 25 is an illustration of one example of the plate information management screen displayed on the monitor. As shown in Fig. 25, the plate information management screen consists of a library ID input region MA-1, a number-of-plates input region MA-2, a plate name input region MA-3, an offset input region MA-4, a plate format input region MA-5, a plate storage location input region MA-6, a plate producer's comment input region MA-7, a project code input region MA-8, a plate producer's input region MA-9, a plate production date input region MA-10, derivative plate name input regions MA-11, derivative plate storage location input regions MA-12, a registration button MA-13, and the like.

The "library ID" is an ID which uniquely identifies cDNA library samples used to produce the plates. The library ID may be associated with, for example, information on the derivation of each sample (e.g., organisms (such as mouse, human, or nematode), a stage (such as eight-week old), a tissue (such as spermary or liver), information on a protocol used for sample preparation (such as a regent quantity, a cloning vector, and a restriction enzyme), information on a producer, information on the production date, and the like.

Further, the "number of plates" (MA-2) is the number of plates to be newly produced and registered. The plates are allocated consecutive ID's based on the "plate names (MA-3) and the "offset" (MA-4). In the example of Fig. 25, for instance, twelve ID's of MEP0001 to MEP0012 are allocated to newly registered plates, respectively.

The "format" (MA-5) is the sequence format of the wells on the plate. In the example of Fig. 25, a standard plate in a 96-well format (A01 to H12) in eight columns (A to H) by twelve rows (01 to 12) is designated. The "storage location" (MA-6) represents an identification name such as a freezer for storing each plate.

As for the library ID, the plate name, the offset, and the format designation, all of or part of these pieces of information can be omitted by the input of the "project code" (MA-8) allocated based on a predetermined rule so as to conceptually include these pieces of information.

The "producer" (MA-9) represents a production operator who produces each actual plate.

The "production date" (MA-10) represents a date when the actual plate is produced.

The "derivative plate names" (MA-11) and "storage locations" (MA-12) can be. appropriately input if there are corresponding derivative plates.

After the experimental operator inputs necessary information on the plate information management screen shown in Fig. 25 at the step S-1, the input of the plate information is completed by clicking on the registration button MA-13 with a mouse. The in situ hybridization analysis management apparatus 100 stores the information input by the operator in a predetermined storage region of the master library database by the processing of the analysis management section.

The analysis management section extracts sequencing step unfinished plate information based on the information on the master library plates newly registered in the master library DB, outputs the information by displaying the analysis progress status management screen on the monitor, and thereby notifies the user (experimental operator) of the information (at a step SA-2).

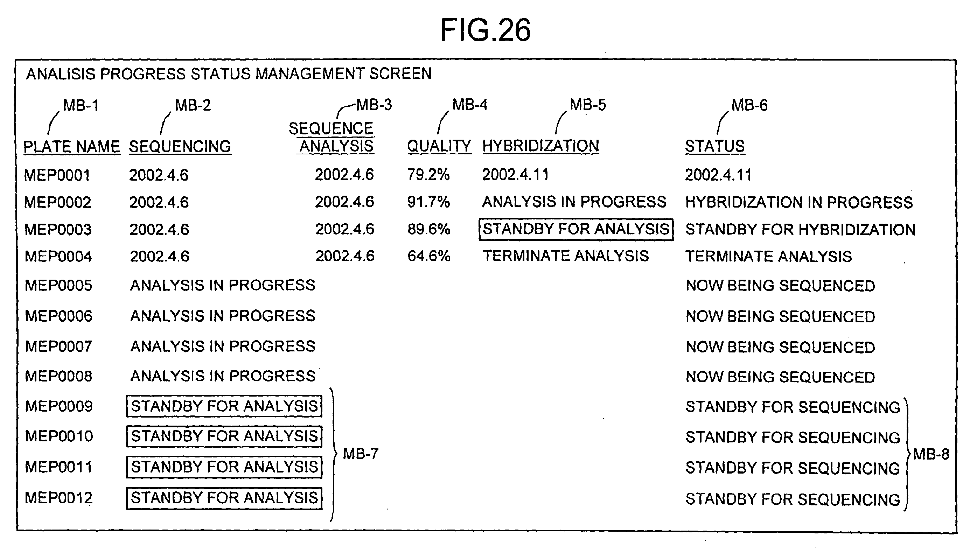

Figs. 26 to 33 illustrate examples of the analysis progress status management screen displayed on the monitor. The analysis progress status management screen represents the analysis progress status of each plate registered at the step SA-1, which status can be used at later steps.

As shown in Figs. 26 to 33, the analysis progress status management screen consists of a plate name space (MB-1), a sequencing space (MB-2), a sequence analysis space (MB-3), a quality space (MB-4), a hybridization space (MB-5), a status space (MB-6), and the like.

On the analysis progress status management screen, the sequencing space (MB-2) shows the progress status of each plate at the sequencing step (2) shown in Fig. 2. If sequencing is finished, the sequencing finished date is displayed in this space.

The sequence analysis space shows the progress status of each plate at the sequence analysis step (3) shown in Fig. 2. If sequencing is finished, the sequencing finished date is displayed in this space.

The quality space shows the yield of each plate completed with the sequence analysis step.

The hybridization space shows the progress status of each plate at the hybridization step (4) shown in Fig. 2. If the hybridization experiment is finished, the hybridization experiment finished date is displayed in this space.

The status space shows the analysis progress status of each plate. If the plate is completed with all the analyses, the analysis finished date is displayed.

As shown in Fig. 26, at the step SA-2, the analysis management section displays "standby for analysis" (MB-7) in the sequencing space of each plate newly registered at the step SA-1. In addition, the analysis management section displays "standby for sequencing" (MB-8) in corresponding status space.

The display of "standby for analysis" (sequencing step unfinished plate information) in the sequencing space is clickable (i.e., characters "standby for analysis" on the monitor screen can be clicked on) (MB-7). In addition, the "standby for analysis" can be displayed with a different color so as to attract operator's attention or display-controlled to be turned on and off.

The operator who performs the experiment (hereinafter, "user") then executes the step P100 shown in Fig. 2 according to the sequencing step unfinished plate information displayed on the monitor. Further, the user executes the step S100 shown in Fig. 2 according to the sequencing step unfinished plate information displayed on the monitor (at a step SA-3).

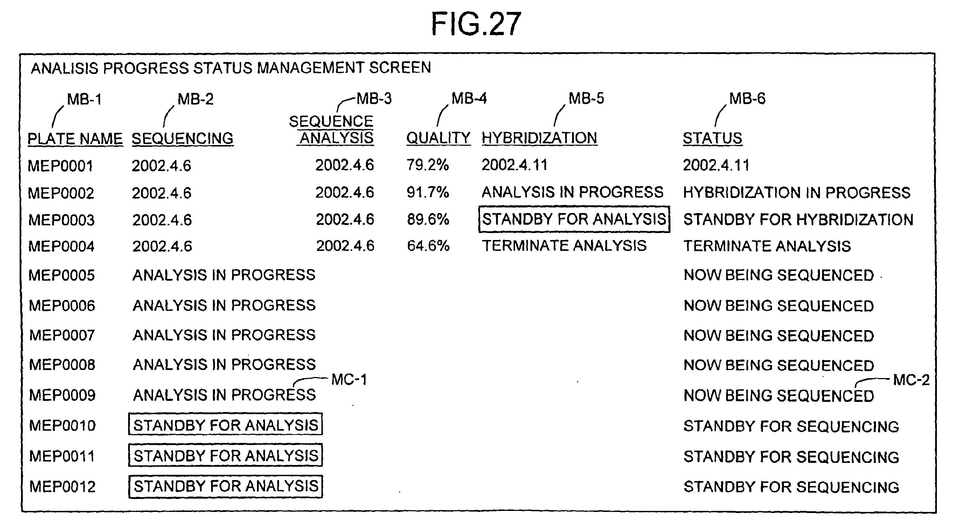

Fig. 27 illustrates the analysis progress status management screen on the monitor that shows an instance in which the user clicks on "standby for analysis" in the sequencing space of the plate MEP0009 shown in Fig. 26 to change the display to "analysis in progress". At the step SA-3, the experimental operator starts the sequencing step for each plate the sequencing step space of which shows "standby for analysis". At this time, the operator clicks on the "standby for analysis" display part by the mouse to thereby notify the analysis management section of the start of an analysis. The analysis management section updates the display of the sequencing space from "standby for analysis" to "analysis in progress" (MC-1), and updates the display of the status space from "standby for sequencing" to "now being sequenced" (MC-2).

At this time, the plate management section may display a master plasmid plate information input screen (similar to the plate information management screen) on the monitor so that the user inputs, through the input device, information (e.g., the plate ID and the storage location of the plate) on each master plasmid plate derived from the master library plate as a result of the step P100 and registers the input information in a predetermined storage region of the master library DB.

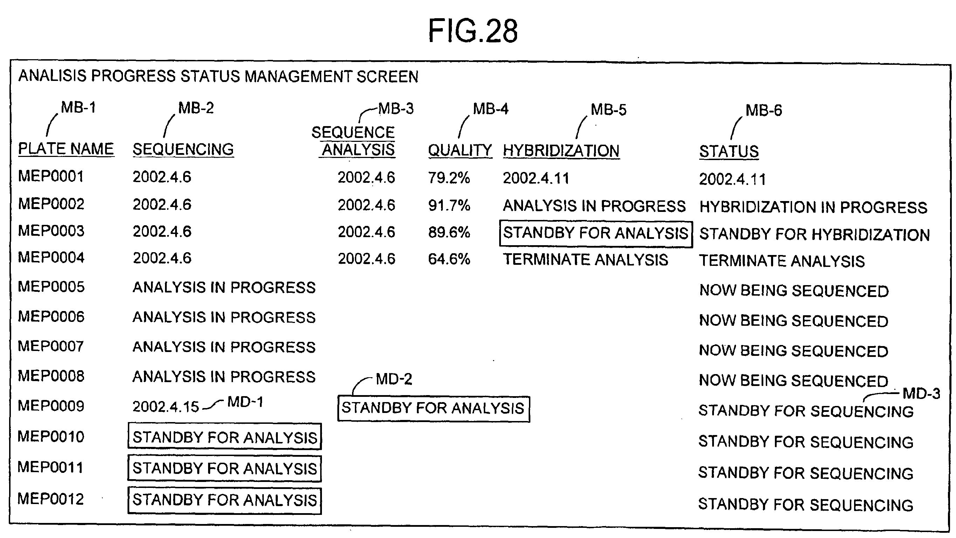

If the sequence management section acquires the read sequence data output from the DNA sequencer at the step S100, and stores the data in a predetermined region of the sequence DB at the step S200, the analysis management section updates the analysis progress status of each related plate in the master library database as shown in Fig. 28 (at a step SA-4). Namely, the analysis management section displays "date" (MD-1) in the sequencing space of the plate, and updates the display of the status space from "now being sequenced" to "standby for analysis" (MD-3).

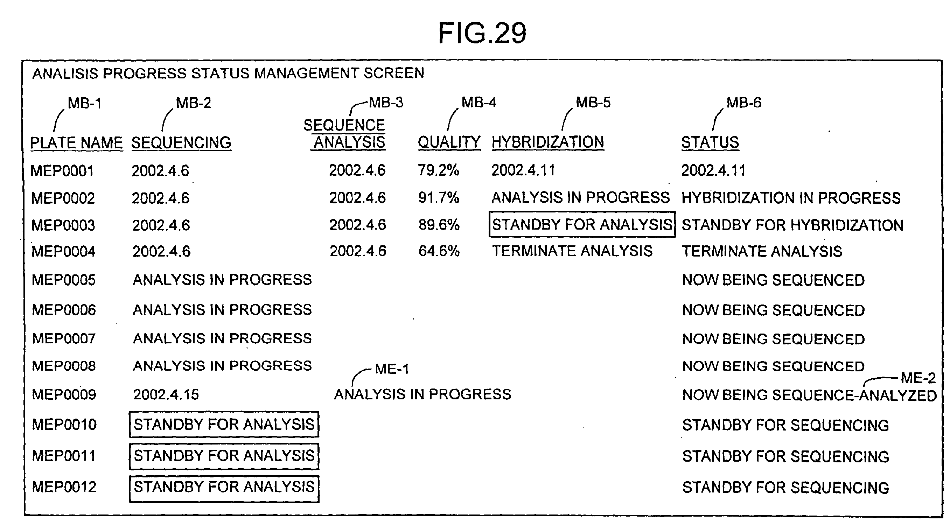

The analysis management section notifies the sequence analysis section of the sequencing finished, sequence analysis step unfinished plates (at a step SA-5). Namely, at the step SA-5, if the analysis management section notifies the sequence analysis section of the sequence analysis step unfinished plates, the analysis management section displays "analysis in progress" (ME-1) in the sequence analysis space of each related plate on the analysis progress status management screen, and updates the display of the status space from "standby for sequence analysis" to "now being sequence-analyzed" (ME-2) as shown in Fig. 29.

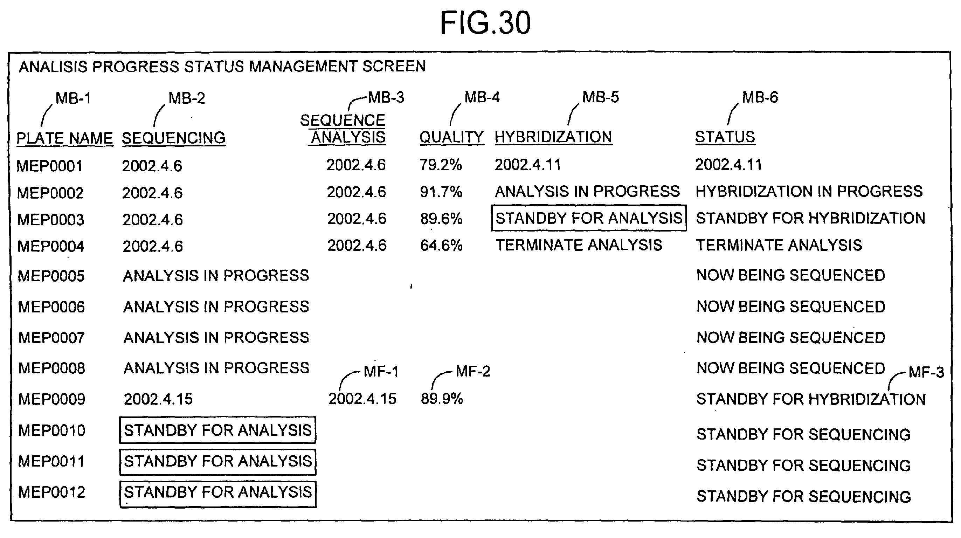

The sequence analysis section acquires sequence data on the sequence analysis step unfinished plates from the sequence DB, executes a series of analysis steps A100 to A300 shown in Fig. 2, and registers execution results in the analysis result DB. The sequence analysis section notifies the plate management section of quality data, which has been known from the analysis, on each related plate. The plate management section updates the analysis progress status of the plate stored in the master library DB (at a step SA-6). At the step SA-6, the analysis management section displays a date (MF-1) in the sequence analysis space of each sequence-analyzed plate and displays quality information (e.g., yield) (MF-2) obtained as a result of the sequence analysis in the quality space of the plate on the analysis progress status management screen as shown in Fig. 30. In addition, the analysis management section updates the display of the status space of the plate from "now being sequence-analyzed" to "standby for hybrid" (MF-3).

The analysis management section outputs information on plates the NG clone rates of which satisfy a certain standard among the sequence analysis step finished, hybridization step unfinished plates to the monitor, and notifies the user (experimental operator) of the information (at a step SA-7).

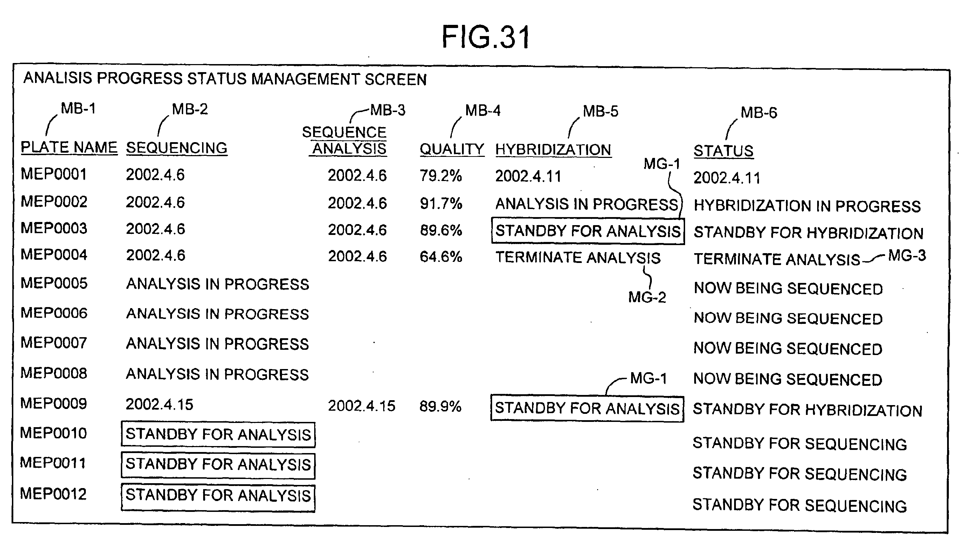

At the step SA-7, the analysis management section displays "standby for hybridization" (MG-1) in the hybridization space of each of the plates the NG clone rates (yield) of which satisfy the certain standard (e.g., 70%) and displays "terminate analysis" (MG-2) in the hybridization space of the plate the NG clone rate (yield) of which does not satisfy the standard and displays "terminate analysis" (MG-3) in the status space thereof, as shown in Fig. 31.

The display of "standby for analysis" (hybridization step unfinished plate information) in the sequencing space is clickable (i.e., characters "standby for analysis" on the monitor screen can be clicked on) (MB-7). In addition, the "standby for analysis" can be displayed with a different color so as to attract operator's attention or display-controlled to be turned on and off.

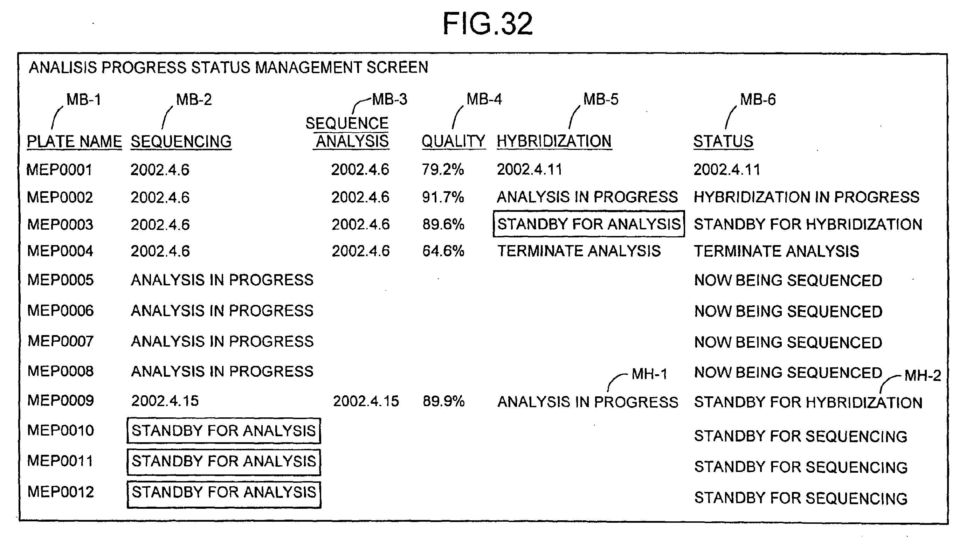

The user (experimental operator) applies the step P200 shown in Fig. 2 to each notified plate and starts executing a hybridization step to the plate (at a step SA-8). At the step SA-8, the experimental operator starts analysis at the hybridization step for each of the plats having "standby for analysis" displayed in the hybridization space of the plate. At this time, by clicking on the displayed part of "standby for analysis" on the monitor shown in Fig. 31, the user notifies the analysis management section of the start of analysis. The analysis management section updates the display of the hybridization space of the plate from "standby for analysis" to "analysis in progress" (MH-1) and updates the display of the status space thereof from "standby for hybridization" to "hybridization in progress" (MH-2) as shown in Fig. 32.

The image management section executes the step H500 of registering the image data obtained as a result of the step H400 in the expression image DB (at a step SA-9).

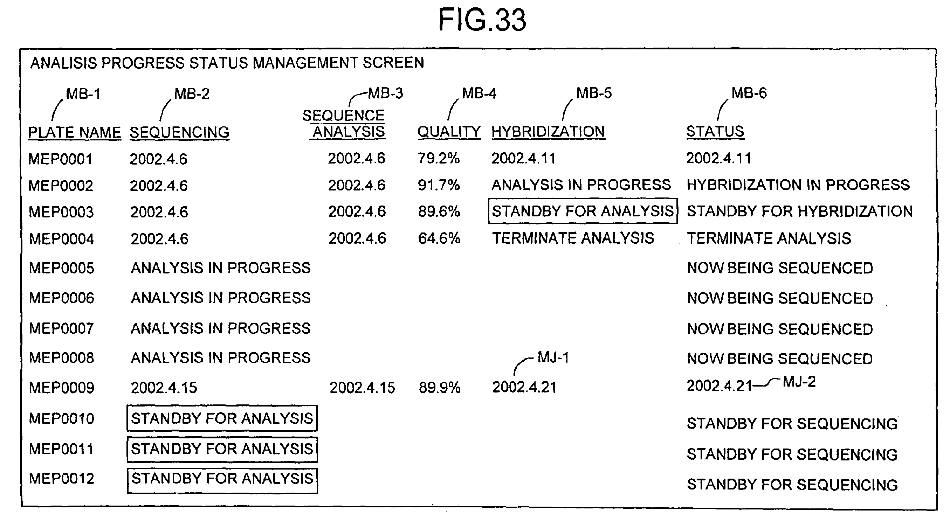

The analysis management section records the end of a series of analyses for each related plate on the analysis progress status of the plate in the master library DB (at a step SA-10). At the step SA-10, the analysis management section displays a date (MJ-1) in the hybridization space of the plate, the image data on which is obtained from the image management section, on the analysis progress status management screen as shown in Fig. 33. At the same time, the analysis management section displays a date (MJ-2) in the status space of the plate.

The synchronous processing of this system is thus finished.

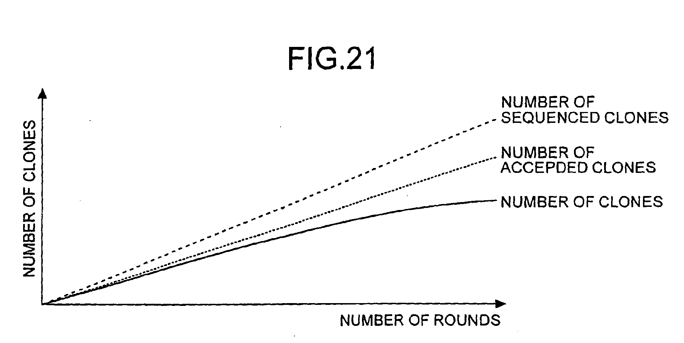

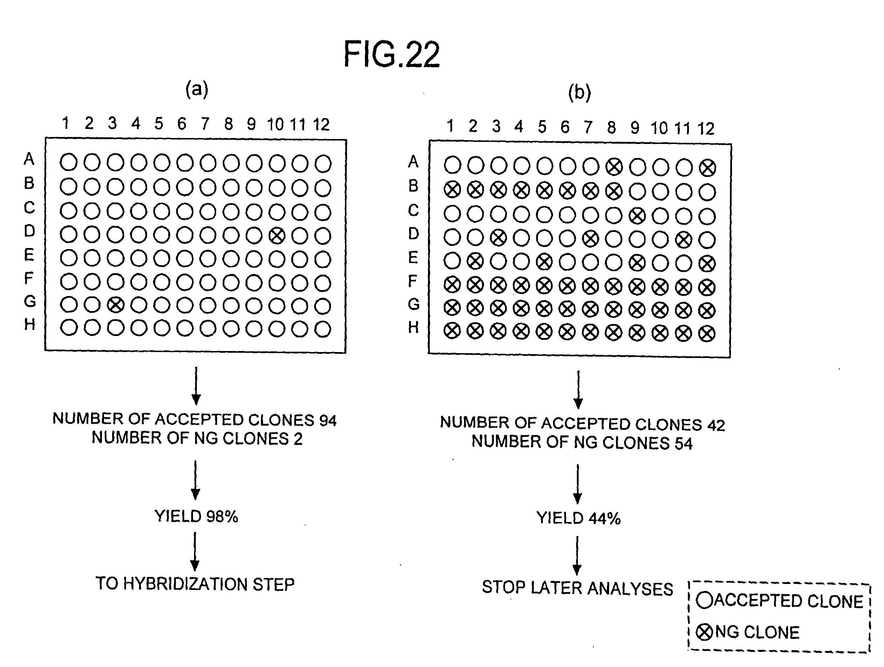

The advantages of the present invention will be explained with reference to Figs. 21 and 22.

A first advantage of the present invention is as follows. Normally, the number of master library plates increases as the master library production step or a part of the step is repeatedly executed. Therefore, it is quite important to this analysis system to determine until when the master library is produced for cost reduction. Since this system constantly manages the analysis status, it is possible to automatically draw a graph as shown in Fig. 21 based on the analysis result DB. The experimental operator can determine the timing of finishing the analyses while monitoring the graph shown in Fig. 21 at appropriate time. (Normally, the number of genes does not increase proportionally with the increase of the number of rounds of the master library production step. Since cost per round is always the same, cost per gene is pushed up, accordingly.)

Another advantage of the present invention is as follows. Normally, at the hybridization step, fixed cost is required per plate. For this reason, if the hybridization step is applied to the plate including many NG clones (unaccepted clones), expression image data cost per clone increases. "Accepted clone" means herein a clone for which an insertion sequence having a sufficient length can be read with sufficient quality as a result of the sequence analysis, otherwise the clone is called "NG clone". In this system, as conceptually shown in Fig. 22, the quality of the mater library is managed. Therefore, the plate with low yield (i.e., the plate in which a certain number or more of NG clones exist) can be detected at a timing as early as possible so as not to proceed the plate to the later analysis steps.

### [The configuration of the system]

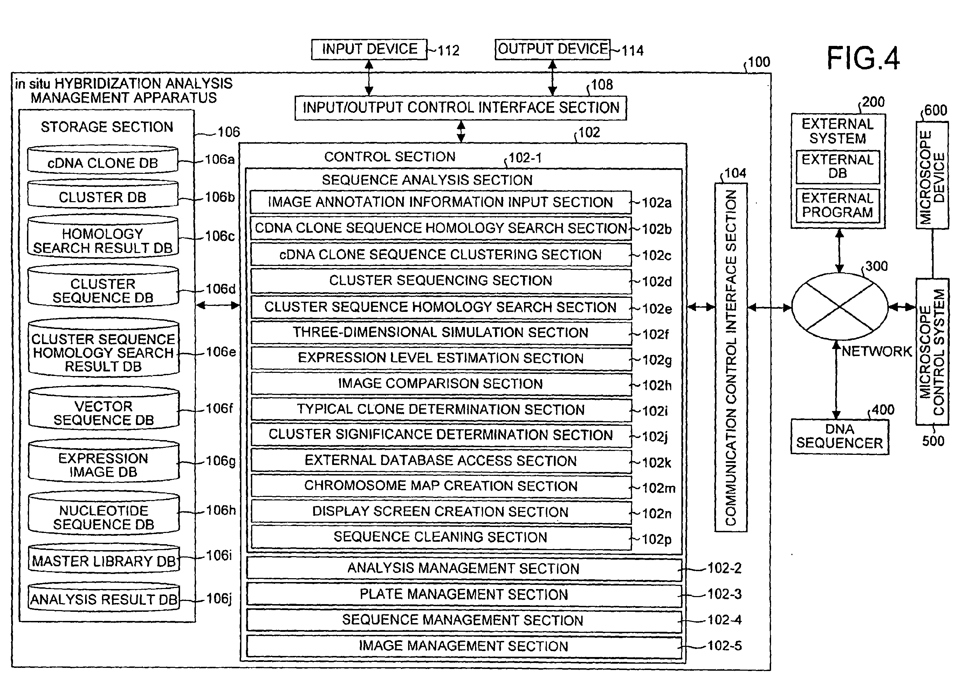

Fig. 4 is a block diagram illustrating one example of the configuration of the system to which the present invention is applied. The system is roughly constituted so that the in situ hybridization analysis management apparatus 100, an external system 200 which provides an external program and the like for an external database related to sequence information and the like, the homology search and the like, a microscope control system 500 which generally controls a microscope device 600, and the DNA sequencer 400 are connected to be communicable with one another through the network 300.

The network 30, for example, is the Internet, and mutually connects the in situ hybridization analysis management section 100 and the external system 200.

The external system 200 is connected to the in situ hybridization analysis management apparatus 100 through the network 300, and functions to provide a website for executing the external databases related to sequence information such as cDNA and the like and the external program such as the homology search program, to the user.

The external system 200 may be constituted as a Web server, an ASP server, or the like. The hardware of the external system 200 may consist of an information processing apparatus and peripheries thereof such as a commercially available workstation or personal computer. Further, the functions of the external system 200 are realized by constituent elements of the hardware such as a CPU, a disk device, a memory device, an input device, an output device, and a communication control device, a program controlling these constituent elements, and the like.

The microscope control system 500 controls the operation of the microscope device 600, takes a microphotograph, and transmits the microphotograph to the in situ hybridization analysis management apparatus 100 through the network 30. Further, the microscope control system 500 receives a control indication command from the in situ hybridization analysis management apparatus 100, and can control the operation of the microscope device 600. The microscope control system 500 may be a commercially available system such as a microscope system (DM-IRE2) (product name) manufactured by Leica Microsystems Incorporated.

The DNA sequencer 400 functions to interpret DNA base sequences. The DNA sequencer 400 may be a commercially available DNA sequencing machine such as ABI3700 (product name) manufactured by Applied Biosystems.

The in situ hybridization analysis management apparatus 100 roughly consists of a control section 102 such as a CPU which generally controls the overall in situ hybridization analysis management apparatus 100, a communication control interface section 104 connected to a communication device (not shown) such as a router connected to a communication line or the like, an input/output control interface section 108 connected to an input device 112 and an output device 114, and a storage section 106 storing various databases and tables. These constituent elements are connected to be communicable with one another through an arbitrary communication path. The in situ hybridization analysis management apparatus 100 is also connected communicably to the network 300 through the communication device such as a router and a wired or wireless communication line such as a dedicated line.

The various databases and tables (a cDNA clone DB 106a to an analysis result DB 106j) stored in the storage section 106 are storage units such as fixed disk devices, and they store various programs, tables, files, databases, webpage files, and the like used in various processings.

Among the constituent elements of the storage section 106, the cDNA clone DB 106a stores cDNA clone identification information for uniquely identifying each cDNA clone and the base sequence (EST sequence) of the cDNA clone while making them correspond to each other.

The cluster DB 106b stores cluster identification information for uniquely identifying each cluster, cDNA clone information on cDNA clones that constitute the cluster, cluster sequence identification information for uniquely identifying a cluster sequence, and cDNA clone identification information on a typical clone while making them correspond to one another.

The homology search result DB 106c stores cDNA clone identification information and the search result of a homology search conducted to the base sequences stored in various base sequence databases for the base sequence of each cDNA clone while making them correspond to each other.

The cluster sequence DB 106d stores cluster sequence identification information and the base sequences of each cluster sequence while making them correspond to each other.

The cluster sequence homology search result database 106e stores cluster sequence identification information and search results of a homology search conducted to base sequences stored in various base sequence database for the base sequences of the cluster sequence while making them correspond to each other.

The vector sequence DB 106f stores cDNA clone identification information and the base sequence of a vector into which cDNA clones are integrated while making them correspond to each other.

The expression image DB 106g stores image identification information for uniquely identifying each image data, cDNA clone identification information, annotation information (e.g., the expression level of each gene, information on an extracted tissue, information on the growth stage or ageing stage of the extracted tissue, information as to whether gene expression is observed, and information on regions in which the gene expression is observed) while making them correspond to one another.

The nucleotide sequence DB 106h stores base sequences such EST sequences and full-length cDNA sequences. The nucleotide sequence DB 106h may be an external base sequence database accessible through the Internet or may be an in-house database produced by copying these databases, storing original sequence information, and adding individual annotation information and the like. The nucleotide DB 106h may store base sequences on (1) a gene which is known in the same or the other organism, (2) a gene which is unknown but a cDNA of which is already acquired, (3) a gene which is unknown but a corresponding genome DNA of which is already acquired, (4) a gene whose location on a chromosome is known, and (5) a gene which is already patented.

The master library DB 106i stores an ID of each plate, a storage location of the plate, an analysis progress status thereof, quality information thereon, the presence/absence of derivative plates thereof, and storage locations of the respective derivative plates, and the like while making them associated with one another.

The analysis result DB 106j stores an ID of each clone, cleaned sequence data on the clone, a homology search result thereof, and the like while making them associated with one another.

The communication control interface section 104 shown in Fig. 4 controls communication between the in situ hybridization analysis management apparatus 100 and the network 300 (or the communication device such as a router). Namely, the communication control interface section 104 functions to communicate data with the other terminals through the communication line. '

The input/output control interface section 108 shown in Fig. 4 controls the input device 112 and the output deice 114. As the output device 114, a monitor (including a home television) or a speaker can be used (note, the term "monitor" will be often used for the output device 114). As the input device 112, a keyboard, a mouse, a microphone, or the like can be used. The monitor realizes a pointing device function in cooperation with the mouse.

The control section 102 shown in Fig. 4 includes an internal memory which stores a control program such as operating system (hereinafter, "OS"), a program specifying various processing procedures or the like, and required data. The control section 102 performs information processings or executing various processings based on these programs. The control section 102 consists of a sequence analysis section 102-1, an analysis management section 102-2, a plate management section 102-3, a sequence management section 102-4, and an image management section 102-5 in terms of functional concept.

The sequence analysis section 102-1 consists of an image annotation information input section 102a, a cDNA clone sequence homology search section 102b, a cDNA clone sequence clustering section 102c, a cluster sequencing section 102d, a cluster sequence homology search section 102e, a three-dimensional simulation section 102f, an expression level estimation section 102g, an image comparison section 102h, a typical clone determination section 102i, a cluster significance determination section 102j, an external database access section 102k, a chromosome map creation section 102m, a display screen creation section 102n, and a sequence cleaning section 102p.

Among the constituent elements of the sequence analysis section 102-1, the image annotation information input section 102a serves as an image data input unit which inputs image data on the expression of a gene and a base sequence input unit which inputs the base sequence of the expressed gene. The cDNA clone sequence homology search section 102b serves as a homology search unit which conducts a homology search to the base sequences input from the base sequence input unit, and extracts homologous base sequences. The cDNA clone sequence clustering section 102c serves as a sequence clustering unit which clusters the base sequences input from the base sequence input unit, and classifies the base sequences into specific clusters, respectively.

The cluster sequencing section 102d serves as a cluster sequencing section which determines a cluster sequence from the base sequences classified into the same class by the sequence clustering unit. The cluster sequence homology search section 102e serves as a cluster sequence homology search unit which conducts a homology search to the cluster sequence determined by the cluster sequencing unit, and which extracts homologous base sequences. The three-dimensional simulation section 102f serves as a three-dimensional image creation unit which creates a three-dimensional image from two or more pieces of image data and an expression level simulation unit which simulates an expression level in the created three-dimensional image based on the expression levels of the image data.

The expression level estimation section 102g serves as an expression level estimation unit which estimates the expression level of a gene in the image data based on one of or both of the image data and the base sequences, and an expression level order sort unit which sorts the display orders of the image data according to the expression level estimated by the expression level estimation unit. The image comparison section 102h serves as an image comparison unit which compares two or more pieces of image data based on at least one of the image data, the base sequences, the expression levels, the information on the extracted tissue, the information on the growth stage or ageing stage of the extracted tissue, the information as to whether the expression of a gene is observed, and information on such region as cells in which the gene expression is observed, and a difference extraction unit which extracts a difference among two or more pieces of image data based on the comparison result of the image comparison unit.

Further, the typical clone determination section 102i serves as a typical clone determination unit which determines a typical clone from the base sequences classified into the same cluster based on at least one of the image data, the base sequences, the expression levels, the information on the extracted tissue, the information on the growth stage or ageing stage of the extracted tissue, the information as to whether the expression of a gene is observed, and the information on such region as cells in which the gene expression is observed.

The cluster significance determination section 102j serves as a cluster significance determination unit which determines the significance of each cluster based on at least one of the homology search result for the cluster sequence, the image data, the base sequences, the expression levels, the information on the extracted tissue, the information on the growth stage or ageing stage of the extracted tissue, the information as to whether the expression of a gene is observed, and the information on such region as cells in which the gene expression is observed. The external database access section 102k serves as an external database access unit which accesses the external database in the external system 200 through the network 300.

The chromosome map creation section 102m serves as a genetic locus specification unit which specifies a genetic locus on a chromosome in which the base sequences exist, a chromosome map creation unit which maps information on the base sequences on the genetic locus of the chromosome and thereby creates a chromosome map, an a chromosome map display unit which displays the chromosome map created by the chromosome map creation unit. The display image creation section 102n serves as a display unit which displays image data, base sequences corresponding to the image data, and analogous base sequences to the corresponding base sequences, and a display unit which displays the image data and the corresponding base sequences for each cluster.

The sequence cleaning section 102p serves as a unit for executing the sequence cleaning processing.

The details of processings performed by these constituent elements will be explained later.

### [The processings by system]

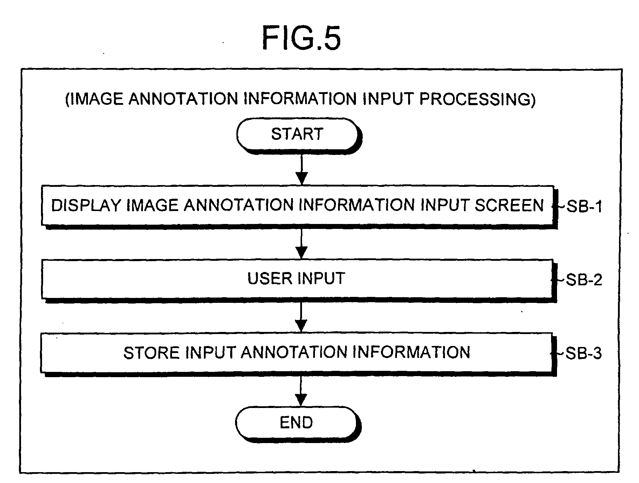

The above system performs, for example, the processings illustrated in Figs. 5 to 13.

### [Image annotation information input processing]

The detail of an image annotation information input processing will be explained with reference to Fig. 5. Fig. 5 is a flow chart illustrating on example of the image annotation information input processing in this embodiment.

The in situ hybridization analysis management apparatus 100 displays an annotation information input screen on the output device 114 by the processing of the image annotation information input section 102a (at a step SB-1).

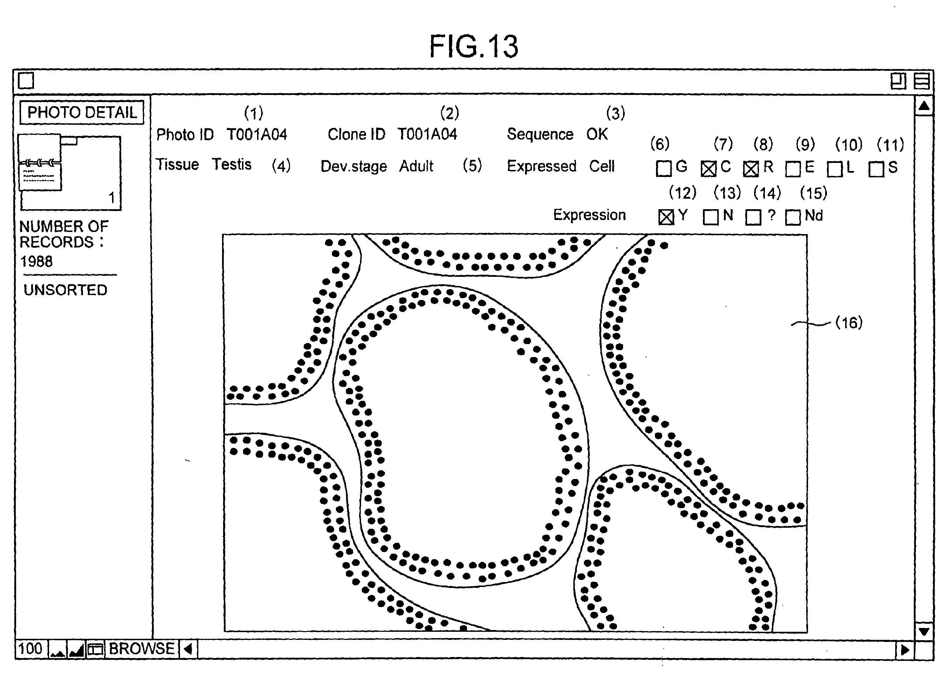

Fig. 13 is an illustration of one example of the annotation information input screen displayed on the monitor. As shown in Fig. 13, the annotation information input screen consists of, for example, an image identification information input region ((1) in Fig. 13), a cDNA clone identification information input region ((2) in Fig. 13), an input region ((3) in Fig. 13) for inputting information as to whether the base sequencing of the cDNA is completed, an input region ((4) in Fig. 13) for inputting the name of a tissue from which a section is extracted, an input region ((5) in Fig. 13) for inputting the growth stage or ageing stage of the organism from which the section is extracted, an input region ((6) to (11) in Fig. 13) for inputting information as to whether the expression of a gene is observed on the section, and an image data display region ((16) in Fig. 13).

If the user checks the annotation information input screen and inputs various pieces of information through the input device 112 (at a step SB-2), the analysis management apparatus 100 stores the input information in a predetermined storage region of the storage section 106 by the processing of the image annotation information input section 102a (at a step SB-3). The image annotation information input processing is thus finished.

### [cDNA clone sequence homology search processing]

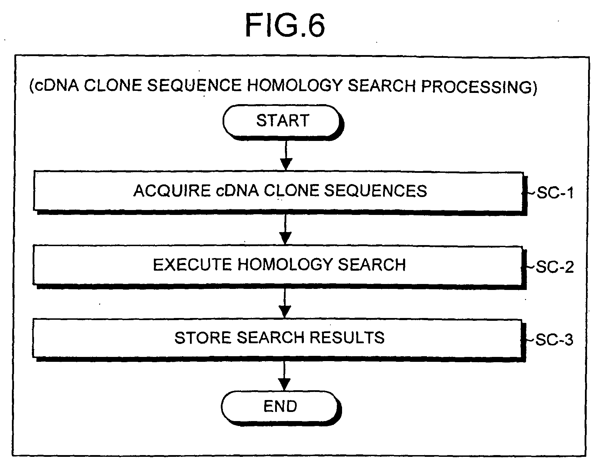

The detail of the cDNA clone sequence homology search processing will be explained with reference to Fig. 6. Fig. 6 is a flow chart illustrating one example of the cDNA clone sequence homology search processing performed by the system in this embodiment.

The in situ hybridization analysis management apparatus 100 accesses the cDNA clone DB 106a and acquires cDNA clone sequences by the processings of the cDNA clone sequence homology search section 102b (at a step SC-1).

The cDNA clone sequence homology search section 102b accesses the nucleotide sequence DB 106h and executes a homology search for the acquired cDNA cone sequences (at a step SC-2). The nucleotide sequence DB 106h accessed by the cDNA clone sequence homology search section 102b includes a DB which stores known genetic sequences on, for example, mammals, a DB which stores known genetic sequences on all organisms, a DB which stores EST sequences (cDNA clone sequence fragments), a DB which stores drafts of genome DNA sequences that are being determined by a genome sequencing project, a DB which stores genome survey sequences (hereinafter, "GSS")(genome DNA clone sequence fragments), a DB which stores sequenced tagged sites (hereinafter, "STS") sequences (sequences mapped on each genome), and a DB which stores already patented genetic sequences.

The cDNA clone sequence homology search section 102b stores homology search results (e.g., homologous sequences, homology scores, each gene name, protein product name of the gene, the ID of the gene in the GenBank DB, the ID of the protein product of the gene in the GenBank DB, information as to length and similarity by which the base sequence of the cDNA is matched with the genetic sequence, and the like) in the homology search result DB 106c (at a step SC-3). The cDNA clone sequence homology search processing is thus finished.

### [Sequence assembly processing]

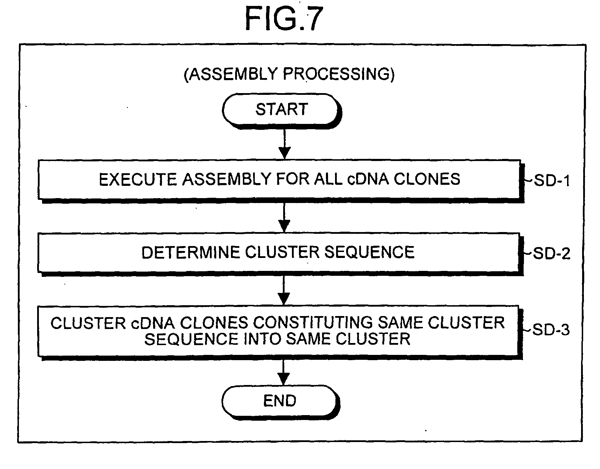

The detail of the sequence assembly processing will be explained with reference to Fig. 7. Fig. 7 is a flow chart illustrating one example of the sequence assembly processing performed by the system in this embodiment.

The in situ hybridization analysis management apparatus 100 accesses the cDNA clone DB 106a and acquires all the cDNA clone sequences by the processings of the cDNA clone sequence clustering section 102c, and assembles consensus sequences using a known sequence assembly software (at a step SD-1).

The cluster sequencing section 102d determines a consensus sequence from a plurality of cDNA clone sequences as a cluster sequence, and stores the cluster sequence in a predetermined storage region of the cluster sequence DB 106d (at a step SD-2).

The cDNA clone sequence clustering section 102c classifies the cDNA clones that constitute the same cluster sequence into the same cluster, and stores the classifies cDNA clones in the cDNA clone DB 106a while associating the respective cDNA clones with the clusters into which the clones are classified, respectively (at a step SD-3). The sequence assembly processing is thus finished.

### [Cluster sequence homology search processing]

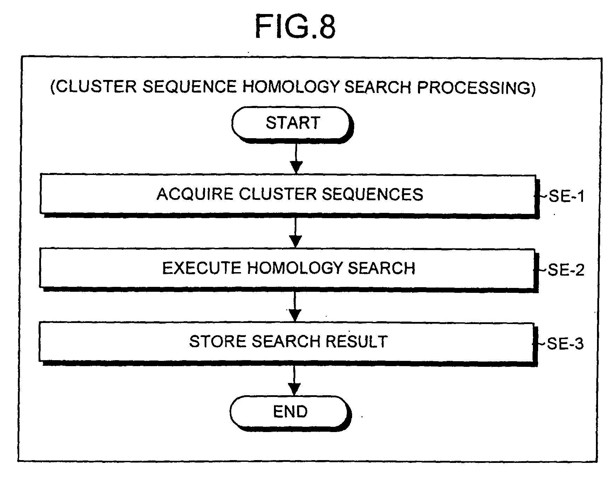

The detail of the cluster sequence homology search processing will be explained with reference to Fig. 8. Fig. 8 is a flow chart illustrating one example of the cluster sequence homology search processing of the system in this embodiment.

The in situ hybridization analysis management apparatus 100 accesses the cluster sequence DB 106d and acquires cluster sequences by the processing of the cluster sequence homology search section 102e (at a step SE-1).

The cluster sequence homology search section 102d accesses the nucleotide sequence DB 106h and executes a homology search to the acquired cluster sequences (at a step SE-2). The nucleotide sequence DB 106h accessed by the cluster sequence homology search section 102d includes the DB which stores known genetic sequences on, for example, mammals, the DB which stores known genetic sequences on all organisms, the DB which stores EST sequences (cDNA clone sequence fragments), the DB which stores drafts of genome DNA sequences that are being determined by a genome sequencing project, the DB which stores GSS (genome DNA clone sequence fragments), the DB which stores STS sequences (sequences mapped on each genome), and the DB which stores already patented genetic sequences.

The cluster sequence homology search section 102e stores homology search results (e.g., homologous sequences, homology scores, each gene name, protein product name of the gene, the ID of the gene in the GenBank DB, the ID of the protein product of the gene in the GenBank DB, information as to length and similarity by which the base sequence of the cDNA is matched with the genetic sequence or information on the proof of the presence of the gene, and the like) in the homology search result DB 106c (at a step SE-3). The cluster sequence homology search processing is thus finished.

### [Three-dimensional simulation processing]

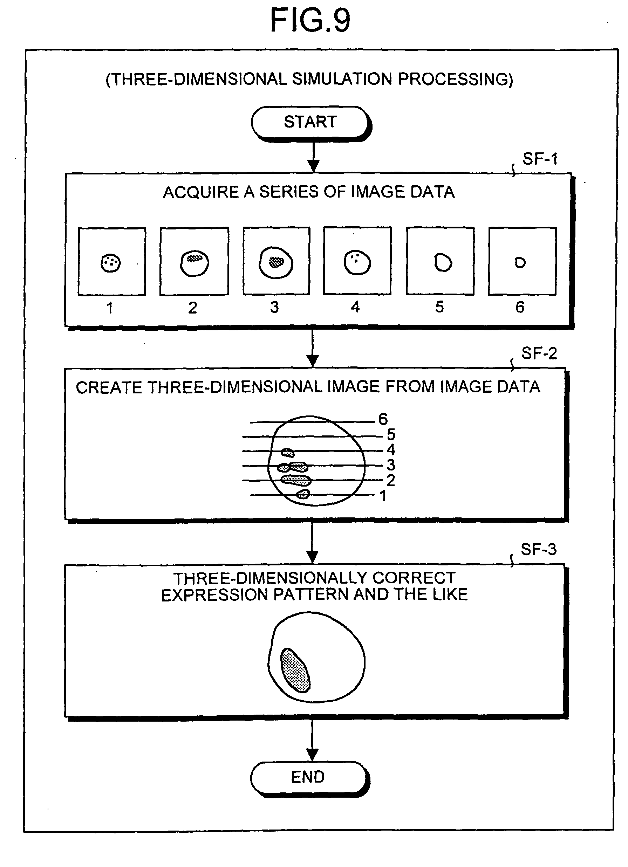

The detail of the three-dimensional simulation processing will be explained with reference to Fig. 9. Fig. 9 is a flow chart illustrating one example of the three-dimensional simulation processing of the system in this embodiment.

If all slices of an organ are tested based on one sequence, the in situ hybridization analysis management apparatus 100 acquires two or more pieces of image data from the expression image DB 106g by the processing of the three-dimensional simulation section 102f (at a step SF-1).

The three-dimensional simulation section 102 creates a three-dimensional (3D) image from the image data using a known 3D display software or the like (at a step SF-2).

The three-dimensional simulation section 102 simulates an expression level in the three-dimensional image based on the expression levels of the respective pieces of image data, three-dimensionally corrects the expression level obtained by analyzing the respective images, and displays the corrected expression level (at a step SF-3). The three-dimensional simulation processing is thus finished.

### [Expression level estimation processing]

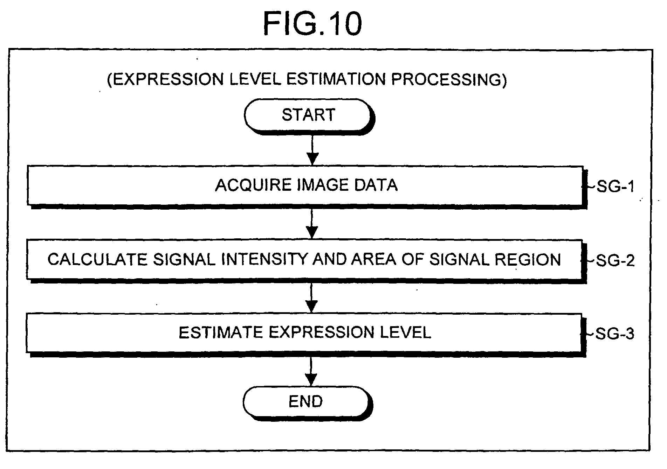

The detail of the expression level estimation processing will be explained with reference to Fig. 10. Fig. 10 is a flow chart illustrating one example of the expression level estimation processing of the system in this embodiment.

The in situ hybridization analysis management apparatus 100 accesses the expression image DB 106g and the cDNA clone DB 106a by the processing of the expression level estimation section 102g, and acquires image data and base sequences (at a step SG-1).

The expression level estimation section 102g estimates the expression level of a gene in the image data based on one of or both of the image data and the base sequences. Namely, the expression level estimation section 102g obtains the signal intensity and the area of an area of signal region of a fluorescent dye or the like in the image data by means of a known image analysis method or the like (at a step SG-2), and estimates the expression level (at a step SG-3).

Further, by using information on not only the image data but also the base sequences, an automatic estimation can be made as follows. If a genomic repeat sequence, for example, is included in the base sequences, the probability of cross-hybridization (occurrence of a hybridization reaction to other mRNA having the same genomic repeat sequence) is high. Therefore, the reliability of the estimated expression level is low. The expression level estimation processing is thus finished.

### [Image comparison processing]

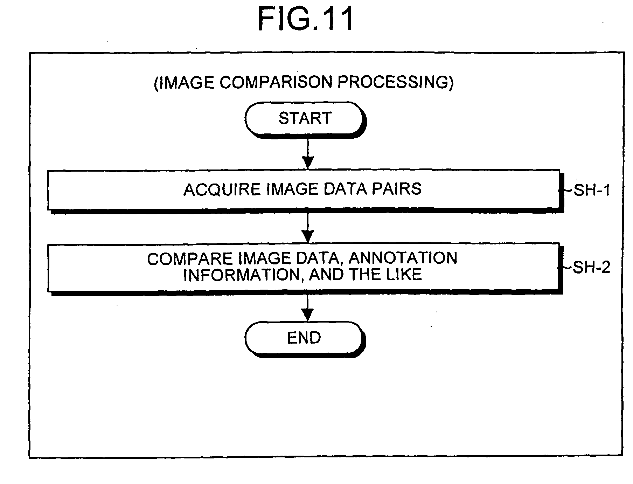

The detail of the image comparison processing will be explained with reference to Fig. 11. Fig. 11 is a flow chart illustrating one example of the image comparison processing of the system in this embodiment.

The in situ hybridization analysis management apparatus 100 accesses the expression image DB 106g and the like by the processing of the image comparison section 102h if comparing, for example, a normal cell with a disease cell, growth stage or ageing stages of the cells at time series, before medication with after medication or the like. The in situ hybridization analysis management apparatus 100 then acquires the image data, base sequences, expression levels, information on the extracted tissue, information on the growth stage or ageing stage of the extracted tissue, information as to whether the expression of a gene is observed, information on such region as cells in which the gene expression is observed, and the like for the comparison target images (at a step SH-1).

The image comparison section 102h compares two or more pieces of data based on the acquired information, and extracts differences among the two or more pieces of image data (at a step SH-2). The image comparison processing is thus finished.

### [Chromosome map creation processing]

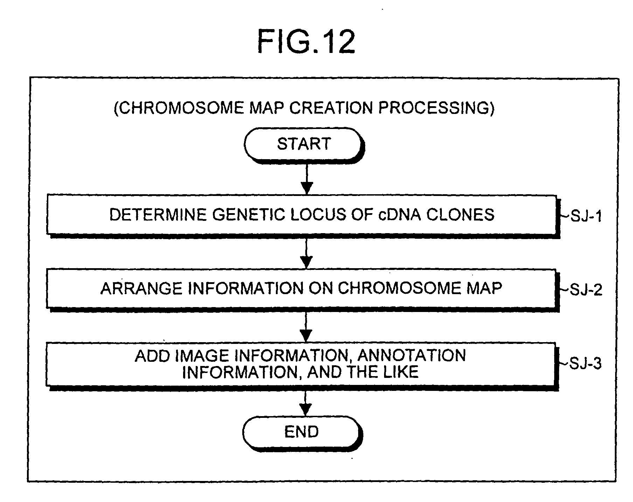

The detail of the chromosome map creation processing will be explained with reference to Fig. 12. Fig. 12 is a flow chart illustrating one example of the chromosome map creation processing of the system in this embodiment.

The in situ hybridization analysis management apparatus 100 accesses the cDNA clone DB 106a or cluster sequence DB 106d, and the nucleotide sequence DB 106h, and thereby specifies the genetic locus of the cDNA clone or the cluster based on base sequence information for which the genetic locus is specified and which is stored in the nucleotide sequence DB 106h by the processing of the chromosome map creation section 102m (at a step SJ-1).

The chromosome map creation section 102m arranges the information on the base sequences (e.g., the image data, base sequences, expression levels, and the extracted tissue, information on the growth stage or ageing stage of the extracted tissue, information as to whether gene expression is observed, and information on regions in which the gene expression is observed) on a chromosome map (by, for example, setting link information or the like), thereby mapping the information on the genetic locus of the chromosome (at a step SJ-2).

If the chromosome map creation section 102m adds information on base sequences and selects a portion (which may be indicated by a specific mark) of the chromosome map corresponding to the genetic locus, the chromosome map creation section 102m displays detailed information on the base sequences (at a step SJ-3). The chromosome map creation processing is thus finished.

### [Exemplary embodiments of the present invention]

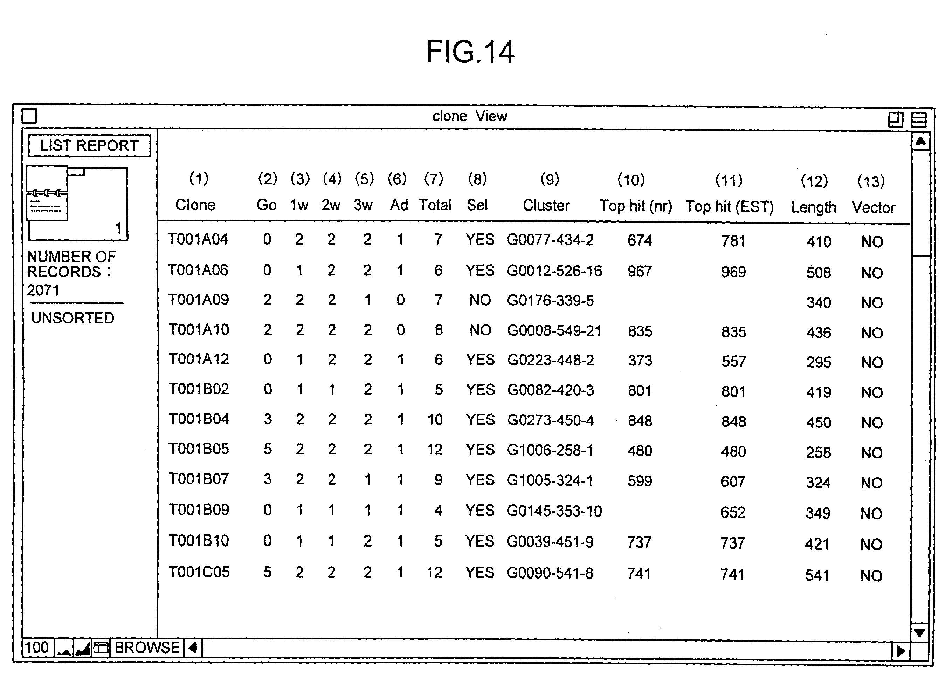

Exemplary embodiments of the present invention constituted as explained above will next be explained with reference to Figs. 14 to 19.

### 1. Viewing of data on each cDNA clone

Fig. 14 illustrates a list report screen displayed if data on each cDNA clone is to be viewed. As shown in Fig. 14, on the list report screen, information on one cDNA clone is displayed in one row. Pieces of information displayed in columns shown in Fig. 14 represent the following information (1) to (13), respectively ((1) to (13) correspond to (1) to (13) shown in Fig. 14).
(1) Display of the ID of a cDNA clone.
(2) Display of the number of in situ hybridization images for gonad at 13.5 dpc of the cDNA clone.
(3) Display of the number of in situ hybridization images for testis of a one-week-old mouse of the cDNA clone.
(4) Display of the number of in situ hybridization images for testis of a two-week-old mouse of the cDNA clone.
(5) Display of the number of in situ hybridization images for testis of a three-week-old mouse of the cDNA clone.
(6) Display of the number of in situ hybridization images for testis of an Adult mouse of the cDNA clone.
(7) Display of the total number of in situ hybridization images of the cDNA clone.
(8) Display of "YES" if the cDNA clone is a typical cDNA clone in a cluster to which the cDNA belongs, and "NO" if not.
(9) Display of the ID of the cluster to which the cDNA belongs.
(10) Display of top hit score as a result of a blast search to a nr (non-redundant nucleotide sequence DB) for the EST sequence of the cDNA clone.
(11) Display of top hit score as a result of a blast search to a dbest (EST sequence DB) for the EST sequence of the cDNA clone.
(12) Display of the length (number of bases) of the EST sequence of the cDNA clone.
(13) Display of "YES" if a vector sequence is included in the EST sequence of the cDNA clone, and "NO" if not.

A detailed report screen will next be described.

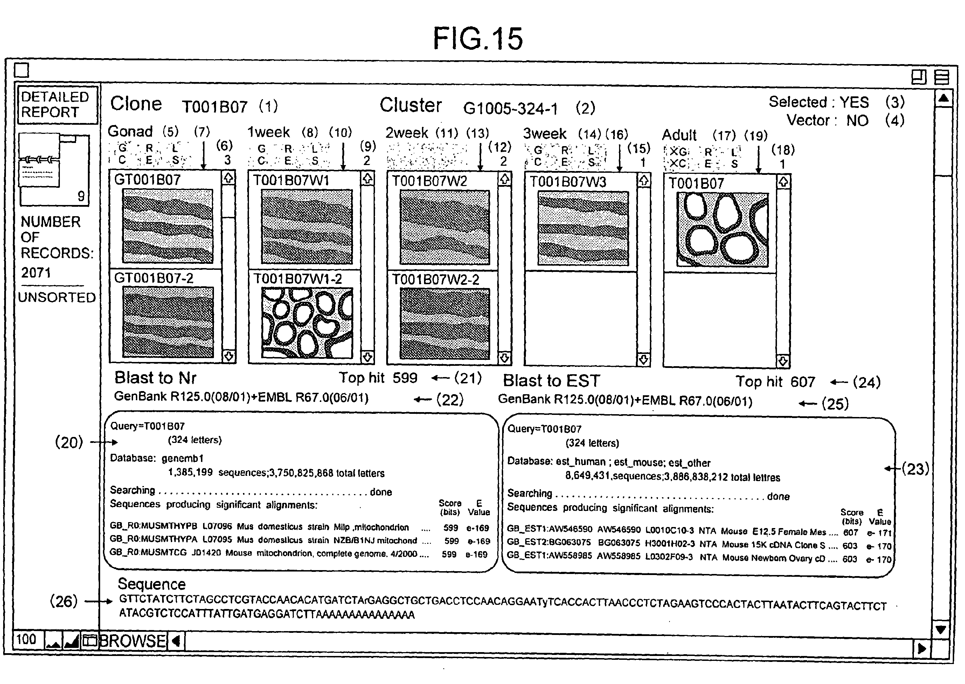

Fig. 15 illustrates the detailed report screen displayed if data on each cDNA clone is to be viewed. As shown in Fig. 15, on the detailed report screen, information on one cDNA clone is displayed on one screen. Pieces of information indicated by items (1) to (26) shown in Fig. 15 are as follows.
(1) Display of the ID of a cDNA clone.
(2) Display of the ID of a cluster to which the cDNA belongs.
(3) Display of "YES" if the cDNA clone is a typical cDNA clone in the cluster to which the cDNA clone belongs, and "NO" if not.
(4) Display "YES" if a vector sequence is included in the EST sequence of the cDNA, and "NO" if not.
(5), (8), (11), (14), and (17) Display of in which cell the expression of the cDNA clone is observed in the gonad at 13.5 dpc, the testis of the one-week-old mouse, the testis of the two-week-old mouse, the testis of the three-week-old mouse, and the testis of the Adult mouse, of the cDNA clone, respectively, by an in situ hybridization experiment. In Fig. 15, symbols G, C, R, E, L, and S denote "Gonia (cell)", "Spermatocyte (cell)", "Round spermatid (cell)", "Elongated spermatid (cell)", "Leydig (cell)", and "Sertoli (cell)", respectively.
(6), (9), (12), (15), (18) Display of the number of in situ hybridization images for the gonad at 13.5 dpc, the testis of the one-week-old mouse, the testis of the two-week-old mouse, the testis of the three-week-old mouse, and the testis of the Adult mouse, of the cDNA clone, respectively.
(7), (10), (13), (16), (19) Display of the in situ hybridization images for the gonad at 13.5 dpc, the testis of the one-week-old mouse, the testis of the two-week-old mouse, the testis of the three-week-old mouse, and the testis of the Adult mouse, of the cDNA clone, respectively. Each image is displayed together with its image ID.
(20) Display of a result of a blast search to the nr for the EST sequence of the cDNA clone.
(21) Display of top hit score as a result of the blast search to the nr for the EST sequence of the cDNA clone.
(22) Display of version information on blast search target nr data for the EST sequence of the cDNA clone.
(23) Display of a result of the blast search to the dbest for the EST sequence of the cDNA clone.
(24) Display of top hit score as a result of the blast search to the dbest for the EST sequence of the cDNA clone.
(25) Display of version information on blast search target dbest data for the EST sequence of the cDNA clone.
(26) Display of the EST sequence of the cDNA clone.

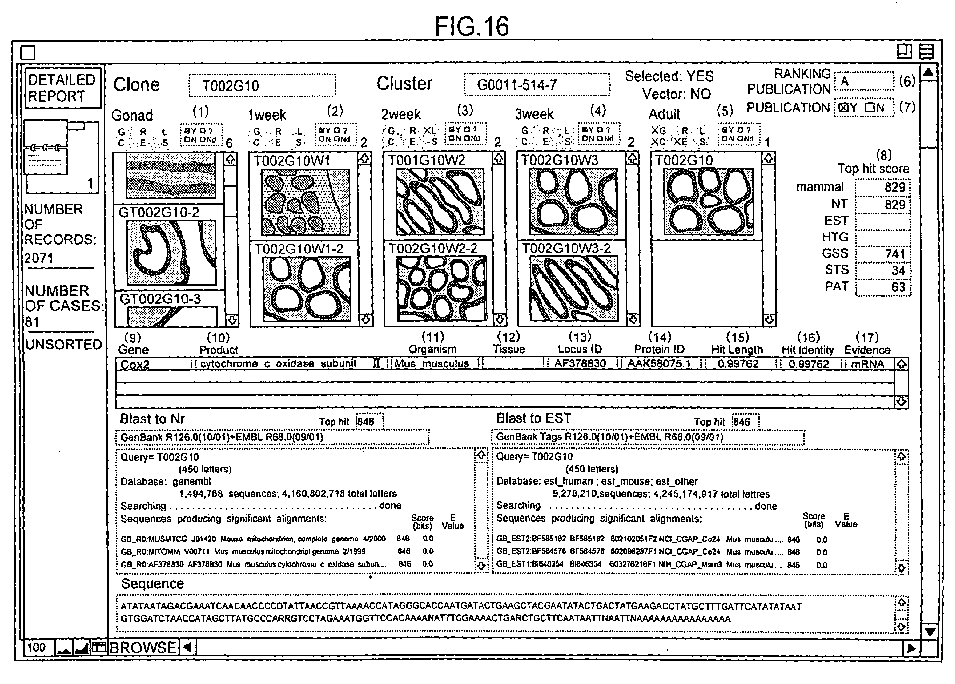

Fig. 16 illustrates another example of the detailed report screen displayed if data on each cDNA clone is to be viewed. Pieces of information indicated by items (1) to (17) shown in Fig. 16 are as follows.
(1) to (5) Display of an expression information field indicated by checkboxes "Y", "N", "?", and "Nd" provided on upper right of an image of the growth stage or ageing stage in each item. This is a field for inputting whether the expression of a gene is observed in the image. Symbol "Y" represents that gene expression is observed, "N" represents that gene expression is not observed, "?" represents that the observation thereof cannot be determined, and "Nd (No data)" represents that no image data is present.
(6) Display of the significance of a cDNA clone.
(7) Display of whether the cDNA clone is inserted in existing databases.
(8) Display of top score as a result of a homology search for the EST sequence of the cDNA clone. This enables the user to check whether the clone is known or unknown. In the item (8), "mammal" denotes a database which stores known genetic sequences about mammals, "NT" denotes a database which stores known genetic sequences about all organisms, "EST" denotes a database which stores EST sequences (sequence fragments of cDNA clones), "high throughput genomic sequencing" (hereinafter, HTG) denotes a database which stores drafts of genome DNA sequences that are being determined by a genome sequencing project, "GSS" denotes a database which stores GSS (sequence fragments of genome DNA clones), "STS" denotes a database which stores STS sequences (sequences mapped on genomes), and "PAT" denotes a database which stores already patented genetic sequences. By viewing this result, the user can check whether the cDNA clone is a gene (mammal, NT) which is known to the same or the other organism, a gene (EST) which is unknown but a cDNA of which is already acquired, a gene (HTG, GSS) which is unknown but a corresponding genome DNA section of which is already acquired, a gene (STS) the position of which on a chromosome is known, or a gene (PAT) which is already patented.
(9) to (17) Display of information on known genes in each field if the cDNA clone is a gene already known. In each item of (9) to (17), symbol "Gene" represents the name of the gene, "Product" represents the name of the protein product of the gene, "Organism" represents an organic species from which the gene is acquired, "Tissue" represent an organ or a tissue from which the gene is acquired, "Locus ID" represents the ID of the gene in the GenBank DB, "Hit Length, Hit Identity" represents the length by which the base sequence of the cDNA coincides with the genetic sequence, and "Evidence" represents display of information (mRNA, DNA or the like; 'mRNA' shows that the presence of an mRNA is confirmed, 'DNA' shows a predicted DNA from the DNA sequence) about the evidence of the presence of the gene.

### 2. Viewing of data on each cluster

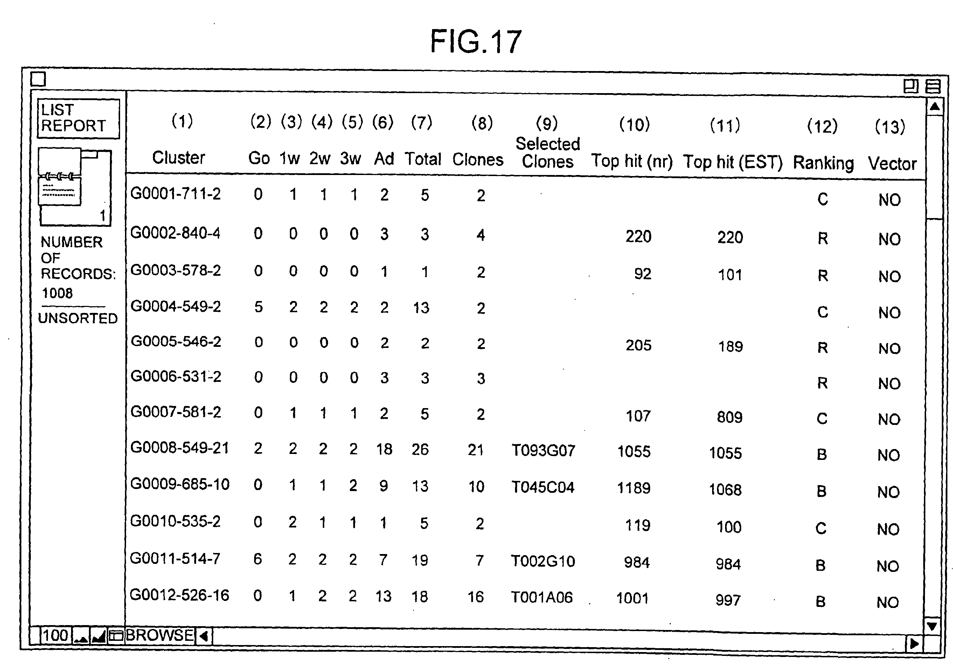

Fig. 17 illustrates a list report screen displayed if data on each cluster is to be viewed. As shown in Fig. 17, on the list report screen, information on one cluster is displayed in one row. Pieces of information displayed in columns shown in Fig. 17 represent the following information (1) to (13), respectively.
(1) Display of the ID of a cDNA clone.
(2) Display of the total number of in situ hybridization images for gonad at 13.5 dpc of all cDNA clones belonging to the cluster.
(3) Display of the total number of in situ hybridization images for testis of a one-week-old mouse of all cDNA clones belonging to the cluster.
(4) Display of the total number of in situ hybridization images for testis of a two-week-old mouse all cDNA clones belonging to the cluster.
(5) Display of the total number of in situ hybridization images for testis of a three-week-old mouse all cDNA clones belonging to the cluster.
(6) Display of the total number of in situ hybridization images for testis of an Adult mouse all cDNA clones belonging to the cluster.
(7) Display of the total number of in situ hybridization images of all cDNA clones belonging to the cluster.
(8) Display of the number of cDNA clones belonging to the cluster.
(9) Display of the ID of a typical cluster in the cluster.
(10) Display of top hit score as a result of a blast search to the nr for the sequence of the cluster.
(11 ) Display of top hit score as a result of a blast search to the dbest for the sequence of the cluster.
(12) Display of the significance of the cluster. Ranked AAA, AA, A, B, ... in descending order of significance.
(13) Display of "YES" if a vector sequence is included in the EST sequence of any one of cDNA clones belonging to the cluster, and "NO" if not.

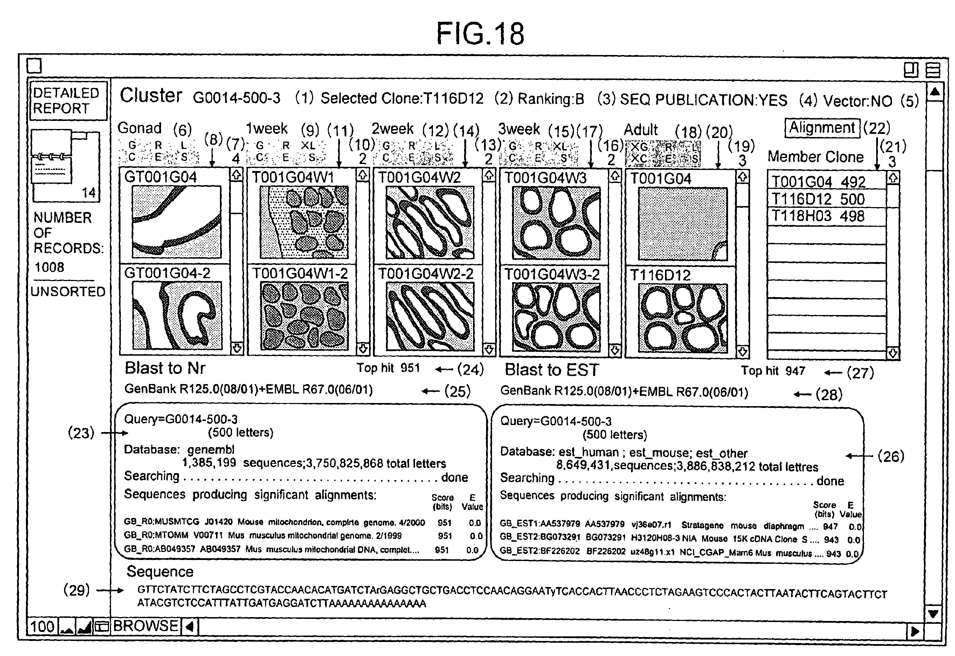

Fig. 18 illustrates the detailed report screen displayed if data on each cluster is to be viewed. As shown in Fig. 18, on the detailed report screen, information on one cluster is displayed on one screen. Pieces of information indicated by items (1) to (29) shown in Fig. 18 are as follows.
(1) Display of the ID of a cluster.
(2) Display of the ID of a typical cDNA clone in the cluster.
(3) Display of the significance of the cluster. Ranked AAA, AA, A, B ... in descending order of significance.
(4) Display of "YES" if the sequence of the cluster and EST sequences of cDNA clones belonging to the cluster are considered to be able to be made open, and "NO" if not.
(5) Display of "YES" if a vector sequence is included in the EST sequence of any one of cDNA clones belonging to the cluster, and "NO" if not.
(6), (9), (12), (15), and (18) Display of in which cell the expression of the cDNA clones belonging to the cluster is observed in the gonad at 13.5 dpc, the testis of the one-week-old mouse, the testis of the two-week-old mouse, the testis of the three-week-old mouse, and the testis of the Adult mouse, of the cDNA clones belonging to the cluster, respectively, by an in situ hybridization experiment. In Fig. 18, symbols G, C, R, E, L, and S denote "Gonia (cell)", "Spermatocyte (cell)", "Round spermatid (cell)", "Elongated spermatid (cell)", "Leydig (cell)", and "Sertoli (cell)", respectively.
(7), (10), (13), (16), and (19) Display of the total number of in situ hybridization images for the gonad at 13.5 dpc, the testis of the one-week-old mouse, the testis of the two-week-old mouse, the testis of the three-week-old mouse, and the testis of the Adult mouse, of the cDNA clones belonging to the cluster, respectively.
(8), (11), (14), (17), and (20) Display of the in situ hybridization images for the gonad at 13.5 dpc, the testis of the one-week-old mouse, the testis of the two-week-old mouse, the testis of the three-week-old mouse, and the testis of the Adult mouse, of the cDNA clones belonging to the cluster, respectively. Each image is displayed together with its image ID.
(21) Display of the number of cDNA clones belonging to the cluster.
(22) Display of ID's of all the cDNA clones belonging to the cluster. For each cDNA clone, the ID of the cDNA clone ID and the EST sequence length (number of bases) of the cDNA clone are displayed.
(23) Display of the result of a blast search to the nr for the sequence of the cluster.
(24) Display of top hit score as a result of the blast search to the nr for the sequence of the cluster.
(25) Display of version information on blast search target nr data for the sequence of the cluster.
(26) Display of the result of the blast search to the dbest for the sequence of the cluster.
(27) Display of top hit score as a result of the blast search to the dbest for the sequence of the cluster.
(28) Display of version information on blast search target dbest data for the sequence of the cluster.
(29) Display of the sequence of the cluster.

### 3. Viewing of chromosome map

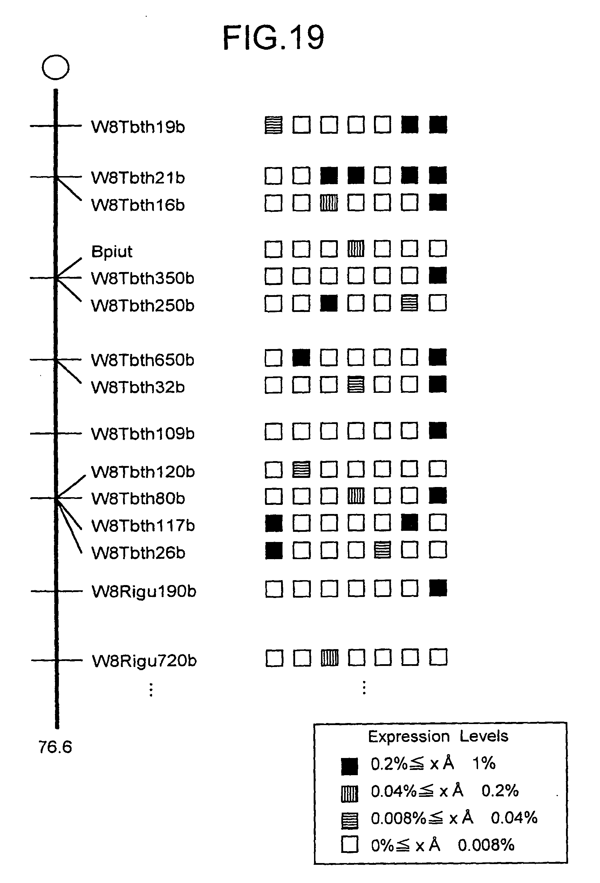

Fig. 19 illustrates a chromosome map display screen displayed if a chromosome map is to be viewed. As shown in Fig. 19, cDNA clones (or clusters) a genetic locus for which has been specified are arranged on the chromosome map. The expression levels of the cDNA clones are listed at time series.

In addition, if each cDNA clone (or cluster name) is selected by clicking on or the like, the detailed report screen of the corresponding cDNA clone or cluster is displayed.

### [Other embodiments of the present invention]

The present invention may be carried out in not only the embodiments explained so far but also various other embodiments within the scope of the technical concept defined by claims which follow.

For example, the instance in which the in situ hybridization analysis management apparatus 100 performs processings in the form of standalone has been explained. Alternatively, the system may be constituted so that each processing is carried out in response to a request from a client terminal provided separately from the in situ hybridization analysis management apparatus 100 and the result of the processing is returned to the client terminal.

Further, all of or part of the processings performed automatically among all the processings as explained in the embodiments can be performed manually, and all of or part of the processings performed manually among all the processings as explained in the embodiment can be performed automatically by a well-known method.

In addition, the processing procedures, control procedures, concrete names, information including various registration data an parameters such as search conditions, examples of screens, and database configurations explained in the specification or shown in the drawings can be arbitrarily changed unless specified otherwise.

Furthermore, the respective constituent elements of the in situ hybridization analysis management apparatus 100 are functionally conceptual elements and not always constituted physically as shown in the drawings.

For example, all of or part of the processing functions of the respective constituent elements of the in situ hybridization analysis management apparatus 100 or the respective devices, particularly the processing functions conducted by the control section 102 can be realized by a central processing unit (hereinafter, "CPU") and programs interpreted and executed by the CPU or can be realized as hardware based on wired logic. The programs are recorded on a recording medium to be explained later and mechanically read by the in situ hybridization analysis management apparatus 100 at need.

Specifically, a computer program for issuing commands to the CPU in cooperation with the OS so as to perform various processings is recorded on the storage section 106 such as a read only memory (hereinafter, "ROM") or a hard disk (hereinafter, "HD"). This computer program is executed by being loaded to a random access memory (hereinafter, "RAM") or the like, and the computer program and the CPU constitutes the control section 102. Alternatively, this computer program may be recorded on an application program server connected to the in situ hybridization analysis management apparatus 100 through the arbitrary network 300. If necessary, the computer program can be downloaded either entirely or partially.

Further, the programs according to the present invention can be stored in a computer readable recording medium. It is assumed herein that examples of the "recording medium" include arbitrary "portable physical mediums" such as a flexible disk, a magneto-optical disk, a ROM, an erasable and programmable ROM (EPROM), an electrically erasable programmable ROM (EEPROM), a CD-ROM, a magnet optical (MO), and a digital versatile disk (DVD), arbitrary "fixed physical mediums" such as a ROM, a RAM and a HD included in a computer system of various types, and "communication mediums" which temporarily hold the programs such as a communication line and a carrier wave used when the programs are transmitted through the network represented by a LAN, a WAN, and the Internet.

Each "program" is a data processing method described in an arbitrary language or description method, and the form of the program may be arbitrary such as source code and binary code. The "program" is not limited to a program constituted unitarily but may be constituted to be distributed as a plurality of modules or libraries or constituted to attain its functions in cooperation with other programs represented by the OS. Concrete configuration of the respective apparatuses shown in the embodiments for reading a recording medium, read procedures, installation procedures after the read procedures, and the like may be well-known configurations and procedures.

The various databases and the like (the cDNA clone DB 106a to the analysis result DB 106j) stored in the storage section 106 are storage units such as memory devices such as RAM and ROM, fixed disk devices such as hard disks, flexible disks, and optical disks. They store various programs, tables, files, databases, webpage files, and the like used in various processings and used to provide websites.

Moreover, the in situ hybridization analysis management apparatus 100 may be realized by connecting a peripheral such as a printer, a monitor, and an image scanner to an information processing apparatus such as an information processing terminal, e.g., a known computer or workstation, and by mounting software (including programs, data, and the like) for realizing the method of the present invention on the information processing apparatus.

Furthermore, the concrete forms of the distribution/integration of the in situ hybridization analysis management apparatus 100 are not limited to those shown in the drawing but the in situ hybridization analysis management apparatus 100 can be constituted to be distributed or integrated physically or functionally in arbitrary units according to various loads and the like. For example, each database can be constituted as a database apparatus independently and part of each processing may be realized using a common gateway interface (CGI).

The network 300 may have a function of mutually connecting the in situ hybridization analysis management apparatus 100 and the external system 200, and include any one of, for example, the Internet, an intranet, a LAN (which may be wired or wireless), a VAN, a personal computer communication network, a public telephone network (which may be analog or digital), a dedicated network (which may be analog or digital), a CATV network, a portable line switching network / portable packet switching network of IMT200, GSM, PDC/PDC-P, and the like, a wireless call network, a local wireless network such as Bluetooth, a PHS network, and a satellite communication network such as CS, BS and ISDB. In short, the system according to the present invention can transmit and receive various pieces of data through an arbitrary network whether wired or wireless.

As explained so far in detail, the present invention inputs image data on the expression of genes, inputs base sequences of the expressed genes (e.g., base sequences of cDNA clones), conducts a homology search of the input base sequences to extract homologous sequences, and displays the image data, the corresponding sequences and the homologous sequences. Therefore, the present invention can provide a gene expression information management apparatus, a gene expression information management method, a program, and a recording medium which can facilitate specifying the genes expressed in the image data.

The present invention conducts the homology search for a base sequence of at least one of: (1) a gene which is known in the same or an other organism; (2) a gene which is unknown but a cDNA of which is already acquired; (3) a gene which is unknown but a corresponding genome DNA of which is already acquired; (4) a gene whose location on a chromosome is known; and (5) a gene which is already patented. Therefore, the present invention can provide a gene expression information management apparatus, a gene expression information management method, a program, and a recording medium which can facilitate specifying the biological significance or the like of the image data.

The present invention inputs image data on the expression of genes, inputs base sequences of the expressed genes (e.g., base sequences of cDNA clones), clusters the input base sequences to classify the base sequences into specific clusters, and displays the image data, the corresponding base sequences, and the homologous sequences to the corresponding base sequences for each cluster. Therefore, the present invention can provide a gene expression information management apparatus, a gene expression information management method, a program, and a recording medium which can collect and classify the base sequences having the same property into the specific cluster by classifying, for example, cDNA (EST sequences) derived from the same mRNA into the same cluster.

The present invention determines a cluster sequence from the base sequences classified into the same cluster, and displays the cluster sequence, the image data, and the corresponding base sequences for each cluster. Therefore, the present invention can provide a gene expression information management apparatus, a gene expression information management method, a program, and a recording medium which can determine and display a base sequence (e.g., a full-length cDNA) created by combining the base sequences belonging to the same cluster as the cluster sequence.

The present invention assembles the base sequences into a consensus sequence, classifies the base sequences constituting the same consensus sequence into the same cluster, and determines the consensus sequence of the cluster as the cluster sequence. Therefore, the present invention can provide a gene expression information management apparatus, a gene expression information management method, a program, and a recording medium which can create a cDNA sequence close to a full-length cDNA sequence from partial cDNA sequences using a sequence assembly technique (for creating a long sequence from short sequence fragments. For example, an overlap between sequence fragments is searched by a multiple sequence alignment method or the like, and the sequence fragments having the overlap are synthesized, whereby a longer sequence is created.)

The present invention conducts a homology search to the determined cluster sequence to extract homologous sequences, and displays the cluster sequence, the homologous sequence, the image data, and the corresponding sequences for each of the cluster. Therefore, the present invention can provide a gene expression information management apparatus, a gene expression information management method, a program, and a recording medium which can facilitate specifying the expressed genes in the image data.

The present invention can provide a gene expression information management apparatus, a gene expression information management method, a program, and a recording medium which can store at least one of information on an extracted tissue, information on a growth stage or an ageing stage of the extracted tissue, information as to whether gene expression is observed, and information on a region in which the gene expression is observed while making the at least one information correspond to the image data, and display at least one of the information on the extracted tissue, the information on the growth stage or the ageing stage of the extracted tissue, the information as to whether the gene expression is observed, and the information on the region in which the gene expression is observed while making the at least one information correspond to the image data.

The present invention estimates expression levels of the genes in the image data based on one of or both of the image data and the base sequences. Therefore, the present invention can provide a gene expression information management apparatus, a gene expression information management method, a program, and a recording medium which can facilitate specifying an expression pattern (a pattern of uniform expression, non-uniform expression or the like).

The present invention sorts display orders of the image data according to the estimated expression levels. Therefore, the present invention can provide a gene expression information management apparatus, a gene expression information management method, a program, and a recording medium which enables the user efficiently check the experimental result.

The preset invention compares two or more pieces of the image data based on at least one of the image data, the base sequences, the expression levels, the information on the extracted tissue, the information on the growth stage or the ageing stage of the extracted tissue, the information as to whether the gene expression is observed, and the information on the region in which the gene expression is observed, and extracts a difference among the two or more pieces of the image data based on a comparison result. Therefore, the present invention can provide a gene expression information management apparatus, a gene expression information management method, a program, and a recording medium which can efficiently extract the difference among the images.

The present invention creates a three-dimensional image from two or more pieces of the image data, and simulates expression levels in the three-dimensional image from the expression levels in the image data. Therefore, the present invention can provide a gene expression information management apparatus, a gene expression information management method, a program, and a recording medium which can, if slices of an organ are all tested based on one sequence, simulate the three-dimensional image of the organ simulated by combining the slice images and which can correct and display the expression level of an mRNA obtained by analyzing each image three-dimensionally.

The present invention determines a typical clone from the base sequences belonging to the same cluster, based on at least one of the image data, the base sequences, the expression levels, the information on the extracted tissue, the information on the growth stage or the ageing stage of the extracted tissue, the information as to whether the gene expression is observed, and the information on the region in which the gene expression is observed. Therefore, the present invention can provide a gene expression information management apparatus, a gene expression information management method, a program, and a recording medium which can select, for example, a clone which can be expected to provide the best experimental data from among the clones derived from the same mRNA and extracted as a typical clone.

The present invention determines the significance of each of the clusters based on at least one of a cluster sequence homology search result, the image data, the base sequences, the expression levels, the information on the extracted tissue, the information on the growth stage or the ageing stage of the extracted tissue, the information as to whether the gene expression is observed, and the information on the region in which the gene expression is observed. Therefore, the present invention can provide a gene expression information management apparatus, a gene expression information management method, a computer program, and a recording medium which can arbitrary determine the significance of each cluster and easily discover the cluster which interests the user based on the information.

The present invention can provide a gene expression information management apparatus, a gene expression information management method, a computer program, and a recording medium which can specify a genetic locus on a chromosome in which the base sequences are present, and create a chromosome map by mapping information (e.g., image data, base sequences, expression levels, information on an extracted tissue, information on the growth stage or ageing stage of the extracted tissue, information as to whether gene expression is observed, and information on a region in which the gene expression is observed) on the base sequences on the genetic locus of the chromosome.

The present invention produces a master library of genetic clones to align the genetic clones on a multiwell plate, reads nucleotide information on the genetic clones produced, performs an analysis based on the nucleotide sequence information read, conducts an in situ hybridization experiment using the genetic clones produced, and one of a specific cell, a specific tissue, and a specific organ, and manages progresses of the other steps according to an analysis result. Therefore, the present invention can provide an in situ hybridization analysis management method which can generally manage the in situ hybridization experiment, efficiently execute the respective steps, and avoid doing over the experiment a'gain or conducting an unnecessary experiment.

The present invention manages master library plate information on master library plates used in an in situ hybridization experiment, outputs the master library plate information managed, acquires base sequence data on genetic clones output from a DNA sequencer, conducts a sequence cleaning to the base sequence data, identifies genes, executes sequence clustering the identified genes, acquires data on expression images picked up at the in situ hybridization experiment using the genetic clones of the base sequence data and one of a specific cell, a specific tissue, and a specific organ, manages the base sequence data and the expression image data while making the base sequence data and the expression image data correspond to each other, manages the progress of at least one of the master library plate information management, the mater library plate information output, the sequence analysis, and the sequence and expression image data management. Therefore, the present invention can provide an in situ hybridization analysis management apparatus which can generally manage the in situ hybridization experiment, efficiently execute the respective steps, and avoid doing over the experiment again or conducting an unnecessary experiment.

Further, the present invention provides a master library database, manages information on the produced master library database, and manages the analysis progress status of each plate on the master library database by one of "sequencing step unfinished", "sequence analysis unfinished", "now being sequence-analyzed", "standby for hybridization", "standby for analysis", "terminate analysis". Therefore, the present invention can provide an in situ hybridization analysis management apparatus which can unitarily manage the analysis progress statuses of the plates.

Moreover, the present invention can provide an in situ hybridization analysis management apparatus which can notify the user of the analysis progress status of each plate and the content of the experiment in detail by displaying the analysis progress status management screen on the monitor.

As explained so far, the gene expression information management apparatus, the gene expression information management method, and the program according to the present invention are quite useful in the field of bioinformatics for managing expression images. In addition, the present invention can be widely carried out in varied industrial fields, particularly in the fields of drug, food, cosmetics, medical treatment, gene expression analysis, and the like, and the present invention is quite useful in these fields.

Moreover, the in situ hybridization analysis management method and the in situ hybridization analysis management apparatus according to the present invention can generally manage image information and genetic information acquired by various gene expression experiments and extract knowledges in full. Therefore, the in situ hybridization analysis management method and the in situ hybridization analysis management apparatus according to the present invention are quite useful in the field of bioinformatics for managing image information and genetic information acquired by various gene expression experiments. In addition, the present invention can be widely carried out in varied industrial fields, particularly in the fields of drug, food, cosmetics, medical treatment, gene expression analysis, and the like, and the present invention is quite useful in these fields.

Although the invention has been described with respect to a specific embodiment for a complete and clear disclosure, the appended claims are not to be thus limited but are to be construed as embodying all modifications and alternative constructions that may occur to one skilled in the art which fairly fall within the basic teaching herein set forth.

### INDUSTRIAL APPLICABILITY

As explained above, the gene expression information management apparatus, the gene expression information management method, and program according to the present invention are considerately effective for a bioinformatics field for managing photomicroscopic image of gene expression analysis. Furthermore the present invention can be widely used and extremely effective for a lot of industrial fields, particularly pharmacy, food, cosmetic, medicine, and gene expression analysis, etc.

The in situ hybridization analysis management method and the in situ hybridization analysis management apparatus according to the present invention can integrally manage the image information and the gene-related information acquired by various gene expressions experiments and extract all the findings without missing any. Therefore the in situ hybridization analysis management method and the in situ hybridization analysis management device according to the present invention are considerately effective for a bioinformatics field for analyzing the image information and the gene-related information acquired by various gene expression experiments. Furthermore the present invention can be widely used and extremely effective for a lot of industrial fields, particularly pharmacy, food, cosmetic, medicine, and gene expression analysis, etc.

## Claims

1. A gene expression information management apparatus comprising:
an image data input unit which inputs pieces of image data on expression of genes;
a base sequence input unit which inputs base sequences of the expressed genes;
a homology search unit which conducts a homology search of the base sequences input by the base sequence input unit, and which extracts homologous sequences; and
a display unit which displays the image data, the base sequences corresponding to the image data, and the homologous sequences to the base sequences.

2. The gene expression information management apparatus according to claim 1, wherein
the homology search unit conducts the homology search for a base sequence of at least one of:
(1) a gene which is known in the same or an other organism;
(2) a gene which is unknown but a cDNA of which is already acquired;
(3) a gene which is unknown but a corresponding genome DNA of which is already acquired;
(4) a gene whose location on a chromosome is known; and
(5) a gene which is already patented.

3. A gene expression information management apparatus comprising:
an image data input unit which inputs pieces of image data on expression of genes;
a base sequence input unit which inputs base sequences of the expressed genes;
a sequence clustering unit which clusters the base sequences input by the base sequence input unit, and which classifies the base sequences into specific clusters; and
a display unit which displays the image data and the base sequences corresponding to the image data for each of the clusters.

4. The gene expression information management apparatus according to claim 3, further comprising:
a cluster sequencing unit which determines a cluster sequence from the base sequences classified into the same cluster by the sequence clustering unit, wherein
the display unit displays the cluster sequence, the image data, and the base sequences corresponding to the image data for each of the clusters.

5. The gene expression information management apparatus according to claim 3, wherein
the sequence clustering unit assembles the base sequences into a consensus sequence, and classifies the base sequences constituting the same consensus sequence into the same cluster, and
the sequence clustering unit determines the consensus sequence of the cluster as the cluster sequence.

6. The gene expression information management apparatus according to claim 4, further comprising:
a cluster sequence homology search unit which conducts a homology search of the cluster sequence determined by the cluster sequencing unit, and which extracts homologous base sequences, wherein
the display unit displays the cluster sequence, the analogous sequence to the cluster sequence, the image data, and the base sequences corresponding to the image data for each of the cluster.

7. The gene expression information management apparatus according to claim 6, wherein
the cluster sequence homology search unit conducts the homology search for a base sequence of at least one of:
(1) a gene which is known in the same or an other organism;
(2) a gene which is unknown but a cDNA of which is already acquired;
(3) a gene which is unknown but a corresponding genome DNA of which is already acquired;
(4) a gene whose location on a chromosome is known; and
(5) a gene which is already patented.

8. The gene expression information management apparatus according to claim 1, further comprising:
an annotation information storage unit which stores at least one of information on an extracted tissue, information on a growth stage or an ageing stage of the extracted tissue, information as to whether gene expression is observed, and information on a region in which the gene expression is observed while making the at least one information correspond to the image data, wherein
the display unit displays at least one of the information on the extracted tissue, the information on the growth stage or the ageing stage of the extracted tissue, the information as to whether the gene expression is observed, and the information on the region in which the gene expression is observed while making the at least one information correspond to the image data.

9. The gene expression information management apparatus according to claim 1, further comprising:
an expression level estimation unit which estimates expression levels of the genes in the image data based on one of or both of the image data and the base sequences.

10. The gene expression information management apparatus according to claim 9, further comprising:
an expression level order sorting unit which sorts display orders of the image data according to the expression levels estimated by the expression level estimation unit.

11. The gene expression information management apparatus according to claim 1, further comprising:
an image comparison unit which compares two or more pieces of the image data based on at least one of the image data, the base sequences, the expression levels, the information on the extracted tissue, the information on the growth stage or the ageing stage of the extracted tissue, the information as to whether the gene expression is observed, and the information on the region in which the gene expression is observed; and
a difference extraction unit which extracts a difference among the two or more pieces of the image data based on a comparison result of the image comparison unit.

12. The gene expression information management apparatus according to claim 1, further comprising:
a three-dimensional image creation unit which creates a three-dimensional image from two or more pieces of the image data; and
an expression level simulation unit which simulates expression levels in the three-dimensional image from the expression levels in the image data.

13. The gene expression information management apparatus according to claim 3, further comprising:
a typical clone determination unit which determines a typical clone from the base sequences belonging to the same cluster, based on at least one of the image data, the base sequences, the expression levels, the information on the extracted tissue, the information on the growth stage or the ageing stage of the extracted tissue, the information as to whether the gene expression is observed, and the information on the region in which the gene expression is observed.

14. The gene expression information management apparatus according to claim 3, further comprising:
a cluster significance determination unit which determines a significance of each of the clusters based on at least one of a cluster sequence homology search result, the image data, the base sequences, the expression levels, the information on the extracted tissue, the information on the growth stage or the ageing stage of the extracted tissue, the information as to whether the gene expression is observed, and the information on the region in which the gene expression is observed.

15. The gene expression information management apparatus according to claim 1, further comprising:
a genetic locus specification unit which specifies a genetic locus on a chromosome in which the base sequences are present;
a chromosome map creation unit which creates a chromosome map by mapping information on the base sequences on the genetic locus of the chromosome; and
a chromosome map display unit which displays the chromosome map created by the chromosome map creation unit.

16. A gene expression information management method comprising:
an image data input step of inputting pieces of image data on expression of genes;
a base sequence input step of inputting base sequences of the expressed genes;
a homology search step of conducting a homology search of the base sequences input at the base sequence input step, and extracting homologous sequences; and
a display step of displaying the image data, the base sequences corresponding to the image data, and the homologous sequences to the base sequences.

17. The gene expression information management method according to claim 16, wherein
at the homology search step, the homology search is conducted for a base sequence of at least one of:
(1) a gene which is known in the same or an other organism;
(2) a gene which is unknown but a cDNA of which is already acquired;
(3) a gene which is unknown but a corresponding genome DNA of which is already acquired;
(4) a gene whose location on a chromosome is known; and
(5) a gene which is already patented.

18. A gene expression information management method comprising:
an image data input step of inputting pieces of image data on expression of genes;
a base sequence input step of inputting base sequences of the expressed genes;
a sequence clustering step of sequence clustering the base sequences input at the base sequence input step, and classifying the base sequences into specific clusters; and
a display step of displaying the image data and the base sequences corresponding to the image data for each of the clusters.

19. The gene expression information management method according to claim 18, further comprising:
a cluster sequencing step of determining a cluster sequence from the base sequences classified into the same cluster at the sequence clustering step, wherein
at the display step, the cluster sequence and the base sequences corresponding to the image data are displayed for each of the clusters.

20. The gene expression information management method according to claim 18, wherein
at the sequence clustering step, the base sequences are assembled into a consensus sequence, the base sequences constituting the same consensus sequence are classified into the same cluster, and
at the sequence clustering step, the consensus sequence of the cluster is determined as the cluster sequence.

21. The gene expression information management method according to claim 19, further comprising:
a cluster sequence homology search step of conducting a homology search of the cluster sequence determined at the cluster sequencing step, and extracting homologous base sequences, wherein
at the display step, the cluster sequence, the analogous sequence to the cluster sequence, and the base sequences corresponding to the image data are displayed for each of the cluster.

22. The gene expression information management method according to claim 21, wherein
at the cluster sequence homology search step, the homology search is conducts for a base sequence of at least one of:
(1) a gene which is known in the same or an other organism;
(2) a gene which is unknown but a cDNA of which is already acquired;
(3) a gene which is unknown but a corresponding genome DNA of which is already acquired;
(4) a gene whose location on a chromosome is known; and
(5) a gene which is already patented.

23. The gene expression information management method according to claim 16, further comprising:
an annotation information storage step of storing at least one of information on an extracted tissue, information on a growth stage or an ageing stage of the extracted tissue, information as to whether gene expression is observed, and information on a region in which the gene expression is observed while making the at least one information correspond to the image data, wherein
at the display step, at least one of the information on the extracted tissue, the information on the growth stage or the ageing stage of the extracted tissue, the information as to whether the gene expression is observed, and the information on the region in which the gene expression is observed is displayed while making the at least one information correspond to the image data.

24. The gene expression information management method according to claim 16, further comprising:
an expression level estimation step of estimating expression levels of the genes in the image data based on one of or both of the image data and the base sequences.

25. The gene expression information management method according to claim 24, further comprising:
an expression level order sorting step of sorting display orders of the image data according to the expression levels estimated at the expression level estimation step.

26. The gene expression information management method according to claim 16, further comprising:
an image comparison step of comparing two or more pieces of the image data based on at least one of the image data, the base sequences, the expression levels, the information on the extracted tissue, the information on the growth stage or the ageing stage of the extracted tissue, the information as to whether the gene expression is observed, and the information on the region in which the gene expression is observed; and
a difference extraction step of extracting a difference among the two or more pieces of the image data based on a comparison result of the image comparison step.

27. The gene expression information management method according to claim 16, further comprising:
a three-dimensional image creation step of creating a three-dimensional image from two or more pieces of the image data; and
an expression level simulation step of simulating expression levels in the three-dimensional image from the expression levels in the image data.

28. The gene expression information management method according to claim 18, further comprising:
a typical clone determination step of determining a typical clone from the base sequences belonging to the same cluster, based on at least one of the image data, the base sequences, the expression levels, the information on the extracted tissue, the information on the growth stage or the ageing stage of the extracted tissue, the information as to whether the gene expression is observed, and the information on the region in which the gene expression is observed.

29. The gene expression information management method according to claim 18, further comprising:
a cluster significance determination step of determining a significance of each of the clusters based on at least one of a cluster sequence homology search result, the image data, the base sequences, the expression levels, the information on the extracted tissue, the information on the growth stage or the ageing stage of the extracted tissue, the information as to whether the gene expression is observed, and the information on the region in which the gene expression is observed.

30. The gene expression information management method according to claim 16, further comprising:
a genetic locus specification step of specifying a genetic locus on a chromosome in which the base sequences are present;
a chromosome map creation step of creating a chromosome map by mapping information on the base sequences on the genetic locus of the chromosome; and
a chromosome map display step of displaying the chromosome map created at the chromosome map creation step.

31. A computer program that makes a computer to execute:
an image data input step of inputting pieces of image data on expression of genes;
a base sequence input step of inputting base sequences of the expressed genes;
a homology search step of conducting a homology search of the base sequences input at the base sequence input step, and extracting homologous sequences; and
a display step of displaying the base sequences corresponding to the image data and the homologous sequences to the base sequences.

32. The computer program according to claim 31, wherein
at the homology search step, the homology search is conducted for a base sequence of at least one of:
(1) a gene which is known in the same or an other organism;
(2) a gene which is unknown but a cDNA of which is already acquired;
(3) a gene which is unknown but a corresponding genome DNA of which is already acquired;
(4) a gene whose on a chromosome is known; and
(5) a gene which is already patented.

33. A computer program that makes a computer to execute:
an image data input step of inputting pieces of image data on expression of genes;
a base sequence input step of inputting base sequences of the expressed genes;
a sequence clustering step of sequence clustering the base sequences input at the base sequence input step, and classifying the base sequences into specific clusters; and
a display step of displaying the image data and the base sequences corresponding to the image data for each of the clusters.

34. The computer program according to claim 33, wherein
the gene expression information management method further comprises a cluster sequencing step of determining a cluster sequence from the base sequences classified into the same cluster at the sequence clustering step, and
at the display step, the cluster sequence and the base sequences corresponding to the image data are displayed for each of the clusters.

35. The computer program according to claim 33, wherein
at the sequence clustering step, the base sequences are assembled into a consensus sequence, the base sequences constituting the same consensus sequence are classified into the same cluster, and
at the sequence clustering step, the consensus sequence of the cluster is determined as the cluster sequence.

36. The computer program according to claim 34, wherein
the gene expression information management method further comprises a cluster sequence homology search step of conducting a homology search of the cluster sequence determined at the cluster sequencing step, and extracting homologous base sequences, and
at the display step, the cluster sequence, the analogous sequence to the cluster sequence, and the base sequences corresponding to the image data are displayed for each of the cluster.

37. The computer program according to claim 36, wherein
at the cluster sequence homology search step, the homology search is conducts for a base sequence of at least one of:
(1) a gene which is known in the same or an other organism;.
(2) a gene which is unknown but a cDNA of which is already acquired;
(3) a gene which is unknown but a corresponding genome DNA of which is already acquired;
(4) a gene whose location on a chromosome is known; and
(5) a gene which is already patented.

38. The computer program according to claim 31, wherein
the gene expression information management method further comprises an annotation information storage step of storing at least one of information on an extracted tissue, information on a growth stage or an ageing stage of the extracted tissue, information as to whether gene expression is observed, and information on a region in which the gene expression is observed while making the at least one information correspond to the image data, and
at the display step, at least one of the information on the extracted tissue, the information on the growth stage or the ageing stage of the extracted tissue, the information as to whether the gene expression is observed, and the information on the region in which the gene expression is observed is displayed while making the at least one information correspond to the image data.

39. The computer program according to claim 31, wherein
the gene expression information management method further comprises an expression level estimation step of estimating expression levels of the genes in the image data based on one of or both of the image data and the base sequences.

40. The computer program according to claim 39, wherein
the gene expression information management method further comprises an expression level order sorting step of sorting display orders of the image data according to the expression levels estimated at the expression level estimation step.

41. The computer program according to claim 31, wherein
the gene expression information management method further comprises:
an image comparison step of comparing two or more pieces of the image data based on at least one of the image data, the base sequences, the expression levels, the information on the extracted tissue, the information on the growth stage or the ageing stage of the extracted tissue, the information as to whether the gene expression is observed, and the information on the region in which the gene expression is observed; and
a difference extraction step of extracting a difference among the two or more pieces of the image data based on a comparison result of the image comparison step.

42. The computer program according to claim 31, wherein
the gene expression information management method further comprises:
a three-dimensional image creation step of creating a three-dimensional image from two or more pieces of the image data; and
an expression level simulation step of simulating expression levels in the three-dimensional image from the expression levels in the image data.

43. The computer program according to claim 33, wherein
the gene expression information management method further comprises a typical clone determination step of determining a typical clone from the base sequences belonging to the same cluster, based on at least one of the image data, the base sequences, the expression levels, the information on the extracted tissue, the information on the growth stage or the ageing stage of the extracted tissue, the information as to whether the gene expression is observed, and the information on the region in which the gene expression is observed.

44. The computer program according to claim 33, wherein
the gene expression information management method further comprises a cluster significance determination step of determining a significance of each of the clusters based on at least one of a cluster sequence homology search result, the image data, the base sequences, the expression levels, the information on the extracted tissue, the information on the growth stage or the ageing stage of the extracted tissue, the information as to whether the gene expression is observed, and the information on the region in which the gene expression is observed.

45. The computer program according to claim 31, wherein
the gene expression information management method further comprises:
a genetic locus specification step of specifying a genetic locus on a chromosome in which the base sequences are present;
a chromosome map creation step of creating a chromosome map by mapping information on the base sequences on the genetic locus of the chromosome; and
a chromosome map display step of displaying the chromosome map created at the chromosome map creation step.

46. An in situ hybridization analysis management method comprising:
a master library production step of producing a master library of genetic clones, and aligning the genetic clones on a multiwell plate;
a sequencing step of reading nucleotide information on the genetic clones produced at the master library production step;
a sequence analysis step of performing an analysis based on the nucleotide sequence information read at the sequencing step;
a hybridization step of conducing an in situ hybridization experiment using the genetic clones produced at the master library step, and one of a specific cell, a specific tissue, and a specific organ; and
a progress management step of managing a progress of steps other than the progress management step according to an analysis result of the sequence analysis step.

47. An in situ hybridization analysis management apparatus comprising:
a master library plate information management unit which manages master library plate information on master library plates used in an in situ hybridization experiment;
a master library plate information output unit which outputs the master library plate information managed by the master library plate information management unit;
a sequence analysis unit which acquires base sequence data on genetic clones output from a DNA sequencer, which conducts a sequence cleaning to the base sequence data, which identifies genes, and which executes sequence clustering the identified genes;
a sequence and expression image data management unit which acquires data on expression images picked up at the in situ hybridization experiment using the genetic clones of the base sequence data and one of a specific cell, a specific tissue, and a specific organ, an which manages the base sequence data and the expression image data while making the base sequence data and the expression image data correspond to each other; and
an analysis management unit which manages at least one of the master library plate information management unit, the mater library plate information output unit, the sequence analysis unit, and the sequence and expression image data management unit.

48. An in situ hybridization apparatus comprising:
a unit which displays a plate information management screen on a monitor for a user so that the user inputs and checks information on produced master library plates through an input device, which registers the information input by the user in a predetermined region of a master library database, and which stores an analysis progress status of each of the master library plates as "sequencing step unfinished";
a unit which extracts the information on the plates each having the analysis progress status of "sequencing step unfinished" based on the information on the master library plates newly registered in the master library database, which displays an analysis progress status management screen on the monitor, and which thereby notifies the user of the extracted plate information;
a unit which acquires sequence data on the plates designated by sequencing step unfinished plate information, the sequence data output from a DNA sequencer, which stores the acquired sequence data in a predetermined storage region of a sequence database, which updates the analysis progress status of each of the designated plates to "sequence analysis step unfinished", and which displays a "date" in a sequencing space and "standby for analysis" in a sequence analysis pace of each of the plates the sequence data of which is obtained on the analysis progress status management screen;
a unit which notifies a sequence analysis section of the plates each having the analysis progress status of "sequence analysis step unfinished", which updates the analysis progress status of each of the plates to "now being sequence-analyzed", which displays "analysis in progress" in the sequence analysis space of the each plate on the analysis progress status management screen, and which updates display of a status space of the each plate from "standby for sequence analysis" to "now being sequence-analyzed";
a unit which causes the sequence analysis section to acquire the sequence data on the plates each having the analysis progress status of "sequence analysis step unfinished" from the sequence database, to execute a sequence analysis processing, to register an execution result in an analysis result database, and to notify a plate management section of quality information on the sequence data, the quality information being made clear as a result of the analysis;
a unit which updates the analysis progress status of each of the plates in the master library database from "now being sequence-analyzed" to "standby for hybridization", which displays on the analysis progress status management screen the date in the sequence analysis space of each of the plates completed with the sequence analysis, which displays on the analysis progress status management screen the quality information obtained as a result of the sequence analysis in a quality space, and which updates the display of the status space of the each plate from "now being sequence-analyzed" to "standby for hybridization";
a unit which causes the plate management section to output, to the analysis progress status management screen, information on the plates an NG clone rate of each of which satisfies a standard among the plates each having the analysis progress status of "sequence analysis step finished" "in situ hybridization step unfinished"; and
unit which displays "standby for analysis" in a hybridization space of the plates the NG clone rate of each of which satisfies the standard on the analysis progress status management screen, and which displays "terminate analysis" and "terminate analysis" in the hybridization space and the status space of each of the plates the NG clone rate of which does not satisfy the standard, respectively, and which updates the analysis progress status of each of the plates the NG clone rate of which does not satisfy the standard from "standby for hybridization" to "terminate analysis".
